# EUROPEAN PATENT APPLICATION

(11) **EP 3 319 058 A1**
(43) Date of publication of application: **09.05.2018**
(21) Application number: 15897146.5
(22) Date of filing: 30.06.2015
(51) Int. Cl.: G08B 21/02

(54) **ANOMALY DETECTION METHOD, ANOMALY DETECTION PROGRAM, AND INFORMATION PROCESSING DEVICE**

(71) Applicant: Fujitsu Limited, Kawasaki-shi, Kanagawa 211-8588 (JP)
(72) Inventor: MATSUOKA, Kenta, Kawasaki-shi Kanagawa 211-8588 (JP); KASAMA, Kouichirou, Kawasaki-shi Kanagawa 211-8588 (JP)
(74) Representative: Haseltine Lake LLP
(86) International application number: PCT/JP2015/068910
(87) International publication number: WO 2017/002219

(57) **Abstract**

An information processing apparatus acquires data indicating a time when a monitored subject is detected to have assumed a predetermined posture, based on an output value from a sensor corresponding to the monitored subject. The information processing apparatus (100) references a storage unit (110) judges whether the time indicated by the acquired data is included in a time period when the monitored subject assumes the predetermined posture. When the time indicated by the acquired data is not included in the time period when the predetermined posture is assumed, the information processing apparatus (100) detects an abnormality of the monitored subject. On the other hand, when the time indicated by the acquired data is included in the time period when the predetermined posture is assumed, the information processing apparatus (100) does not detect an abnormality of the monitored subject.

## Description

### TECHNICAL FIELD

The present invention relates to an abnormality detection method, an abnormality detection program, and an information processing apparatus.

### BACKGROUND ART

In an existing service, as a part of a monitoring activity for an older adult, etc., a built-in sensor in a pendant, etc. worn by a user detects a falling of the user and notifies a support center.

Related prior arts include a technique of determining whether a behavior of an observed person is abnormal, based on behavior data of the observed person, reference data used for evaluating the behavior of the observed person, and area data acquired by storing results of detection of an area in which a person is present, for example.

Japanese Laid-Open Patent Publication No. 2005-327134

### DISCLOSURE OF INVENTION

### PROBLEM TO BE SOLVED BY THE INVENTION

However, with conventional techniques, an abnormality such as a falling of an older adult may be falsely detected. For example, when a user wearing a pendant, etc. with a built-in sensor that detects falling lies down at bedtime, etc., falling may be detected falsely even though the user is not falling.

According to one aspect, an object of the present invention is to provide an abnormality detection method, an abnormality detection program, and an information processing apparatus that prevent false detection of an abnormality of a monitored subject.

### MEANS FOR SOLVING PROBLEM

According to one aspect of the present invention, an abnormality detection method, an abnormality detection program, and an information processing apparatus are proposed that acquires data indicating a time when a monitored subject is detected to have assumed a predetermined posture, based on an output value from a sensor corresponding to the monitored subject; and reference a storage unit storing information identifying a time period when the monitored subject assumes the predetermined posture and detect an abnormality of the monitored subject when the time indicated by the acquired data is not included in the time period.

### EFFECT OF THE INVENTION

According to an aspect of the present invention, false detection of an abnormality of a monitored subject may be prevented.

### BRIEF DESCRIPTION OF DRAWINGS

FIG. 1 is an explanatory diagram of an example of an abnormality detection method according to an embodiment;
FIG. 2 is an explanatory diagram of a system configuration example of an abnormality detection system 200;
FIG. 3 is a block diagram of a hardware configuration example of a server 201;
FIG. 4 is a block diagram of a hardware configuration example of a wearable terminal 202;
FIG. 5 is an explanatory diagram of an example of storage contents of a monitored-subject DB 220;
FIG. 6 is an explanatory diagram of a specific example of behavior state data;
FIG. 7 is an explanatory diagram of an example of storage contents of a living activity pattern occurrence rate DB 240;
FIG. 8 is a block diagram of a functional configuration example of the wearable terminal 202;
FIG. 9 is a block diagram of a functional configuration example of the server 201;
FIG. 10 is an explanatory diagram of a specific example of abnormality notification information;
FIG. 11 is a flowchart of an example of an upload process procedure of the wearable terminal 202;
FIG. 12 is a flowchart of an example of a specific process procedure of a posture determination process;
FIGs. 13A and 13B are flowcharts of an example of a specific process procedure of a movement-type determination process;
FIG. 14 is a flowchart of an example of a specific process procedure of a vital-sign analysis process;
FIG. 15 is a flowchart of an example of a specific process procedure of a surrounding-environment estimation process;
FIG. 16 is a flowchart of an example of a specific process procedure of a position estimation process;
FIG. 17 is a flowchart of an example of a specific process procedure of a sound analysis process;
FIG. 18 is a flowchart of an example of an abnormality detection process procedure of the server 201; and
FIG. 19 is a flowchart of an example of a specific process procedure of a falling determination process.

### BEST MODE(S) FOR CARRYING OUT THE INVENTION

Embodiments of an abnormality detection method, an abnormality detection program, and an information processing apparatus according to the present invention will be described in detail with reference to the accompanying drawings.

### (Example of Abnormality Detection Method)

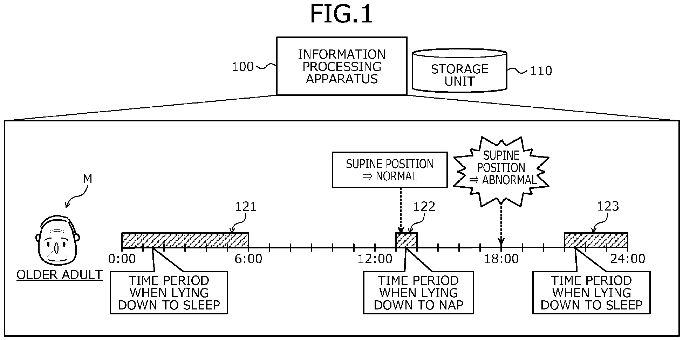

FIG. 1 is an explanatory diagram of an example of an abnormality detection method according to an embodiment. In FIG. 1, an information processing apparatus 100 is a computer that detects an abnormality of a monitored subject. The monitored subject is a person (monitored person) or an object (monitored object) to be monitored. The monitored person is, for example, an older adult, a child, a worker working under a severe environment. The monitored object is, for example, a signboard placed at a store front, material and equipment placed on a construction site, etc.

The information processing apparatus 100 may be applied to a server capable of communicating with a terminal device attached to a monitored subject and detecting the posture of the monitored subject, for example. Alternatively, the information processing apparatus 100 may be applied to a terminal device that is attached to a monitored subject and detects the posture of the monitored subject, for example.

When an older adult, etc. has fallen down, the person may be unable to move due to injury or loss of consciousness, and therefore, it is important that a family member, etc. notice and deal with the situation as soon as possible. If a worker has fallen down in the summer or at site with a poor footing, the worker may be unable to move due to heatstroke or injury and therefore, it is important that a site supervisor, etc. notice and deal with the situation as soon as possible.

A signboard placed at a store front for advertising may fall down due to strong wind or may contact a passer-by. The fallen signboard cannot fulfill the role of advertising and leads to a poor image of the store. Therefore, it is important that an employee, etc. notices and deals with the situation as soon as possible.

Materials and equipment at a construction site, etc. may fall down due to strong winds. If the material or equipment has fallen down, a person who happens to be at the site may be injured and become unable to move and further accidents may occur. Therefore, it is important that employee, etc. notice and deal with the situation as soon as possible.

Thus, for example, it is conceivable that a terminal device with a built-in sensor for detecting an abnormality such as falling is attached to a monitored subject and when an abnormality is detected, a monitoring person is notified. However, if the monitored subject performs a motion similar to a motion at the time of an abnormality such as a falling motion, this may be detected falsely as an abnormal state even though the monitored subject is in a normal state.

For example, when a motion similar to a falling motion is performed by, for example, an older adult lying down at bedtime, etc. or a worker lying down during a break, etc., this behavior may be detected falsely as falling even though the subject is not falling. When a signboard placed at the store front is laid down before being putting away, this action may be detected falsely as falling even though the signboard has been laid down intentionally. If materials or equipment at a construction site are laid down before use, this action may be detected falsely as falling even though the materials or equipment have been laid down intentionally.

The motion of an older adult lying down at bedtime, etc. or a worker lying down during a break, etc. is often habitually performed during a time period that is predetermined to some degree. The motion of laying down a signboard placed at the store front before putting the signboard away or laying down equipment at a construction site before use is often performed during a time period that is predetermined to some degree.

Therefore, the embodiment will be described in terms of an abnormality detection method for preventing false detection of an abnormality of a monitored subject by utilizing the fact that a motion similar to a motion at the time of an abnormality such as a falling is often habitually performed during a time period that is predetermined to some degree. A processing example of the information processing apparatus 100 will hereinafter be described.
(1) The information processing apparatus 100 acquires data indicative of a time when a monitored subject is detected to have assumed a predetermined posture according to an output value from a sensor corresponding to the monitored subject. The sensor corresponding to the monitored subject may be any sensor capable of detecting the posture of the monitored subject and is an acceleration sensor, a gyro sensor, or an atmospheric pressure sensor, for example. The sensor corresponding to the monitored subject may be included in, for example, a terminal device attached to the monitored subject or may directly be attached to the monitored subject.
   The predetermined posture is a posture set according to what kind of abnormality is to be detected of the monitored subject and is set to, for example, the posture when a motion similar to the motion at the time of an abnormality is performed. For example, when a falling of the monitored subject is detected, the predetermined posture is set to a posture when a motion similar to a falling motion is performed.
   In the description of the example of FIG. 1, the monitored subject is an "older adult M", and a "falling" of the monitoring subject is detected. In this description, the predetermined posture is set to a "supine position", which is a posture when the older adult M performs a motion similar to a falling motion such as lying down.
(2) The information processing apparatus 100 refers to a storage unit 110 to judge whether the time indicated by the acquired data is included during a time period when the predetermined posture is assumed. The storage unit 110 is a storage apparatus storing information identifying the time period when the predetermined posture is assumed.
   The time period when the predetermined posture is assumed may manually be set with consideration of a past behavior pattern of the monitored subject, for example. Alternatively, the information processing apparatus 100 may accumulate data indicative of the posture of the monitored subject and the time when the posture is detected, and may statistically analyze the behavior pattern from the accumulated data so as to identify the time period when the predetermined posture is assumed.
   In the example of FIG. 1, time periods when the older adult M assumes the posture of "supine position" are set as a time period 121 from 0 o'clock to 6 o'clock, a time period 122 from 13 o'clock to 14 o'clock, and a time period 123 from 21 o'clock to 23 o'clock. The time periods 121, 123 are the time periods when the older adult M lies down to sleep. The time period 122 is the time period when the older adult M lies down for a nap.
(3) The information processing apparatus 100 detects an abnormality of the monitored subject if the time indicated by the acquired data is not included in the time period when the predetermined posture is assumed. In contrast, the information processing apparatus 100 does not detect an abnormality of the monitored subject if the time indicated by the acquired data is included in the time period when the predetermined posture is assumed.

In the example of FIG. 1, the information processing apparatus 100 detects the "falling" of the older adult M if the time indicated by the acquired data is not included in any of the time periods 121 to 123. For example, when the time indicated by the acquired data is "18:00", the time is not included in any of the time periods 121 to 123 and, therefore, the "falling" of the older adult M is detected.

On the other hand, the information processing apparatus 100 does not detect the "falling" of the older adult M when the time indicated by the acquired data is included in any of the time periods 121 to 123. For example, when the time indicated by the acquired data is "13:00", the time is included in the time period 122 and therefore, "falling" of the older adult M is not detected.

As described above, the information processing apparatus 100 may detect the "falling" of the older adult M if none of the time periods 121 to 123 includes the time when the older adult M is detected to have assumed the posture of "supine position" according to the output value of the sensor corresponding to the older adult M.

As a result, if the time of detection of the older adult M assuming the posture of "supine position" does not match the time when the older adult M habitually assumes the posture of "supine position", the "falling" of the older adult may be detected, so that the older adult M lying down for sleep, etc. may be prevented from being falsely detected as "falling". Consequently, excessive alarms to a monitoring person such as a family member may be suppressed to reduce the burden of the monitoring person.

Although the "older adult M" is described as an example of the monitored subject in the example of FIG. 1, the "falling" of a monitored object such as a signboard may also be detected. For example, the time of detection of the signboard in a position of "being laid down" does not match the time when the signboard is habitually in a position of being laid down, the "falling" of the signboard may be detected, so that the signboard being laid down before being put away may be prevented from being falsely detected as "falling".

In the example of FIG. 1, the case of detecting the "falling" as an abnormality of the monitored subject has been described as an example; however, the present invention is not limited hereto. For example, the older adult M suffering from dementia may suddenly wander and go missing even if the person is usually in a bedridden state. When such "wandering" of the older adult M is to be detected, for example, the predetermined posture may be set to a "standing position" that is a posture when a motion similar to a wandering motion (e.g., walking) is performed. A time period when the monitored subject to assumes the posture of "standing position" is set to, for example, a time period when the person is taken for a bath or on walk by a caregiver. In this case, for example, the information processing apparatus 100 detects the "wandering" of the older adult M if the set time period does not include the time when the older adult M is detected to have assumed the posture of "standing position".

As a result, if the time of detection of the older adult M assuming the posture of "standing position" does not match the time when the older adult M habitually assumes the posture "standing position", the "wandering" of the older adult may be detected, so that the older adult M standing up for a walk, etc. may be prevented from being falsely detected as "wandering".

### (System Configuration Example of Abnormality Detection System 200)

A system configuration example of an abnormality detection system 200 according to the embodiment will be described. In the following description of the example, the information processing apparatus 100 depicted in FIG. 1 is applied to a server 201 of the abnormality detection system 200. An "older adult" is taken as an example of the "monitored subject" in the description.

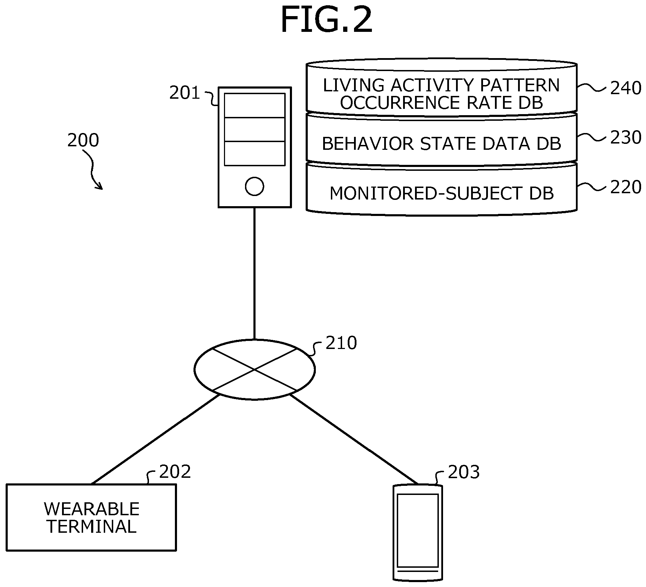

FIG. 2 is an explanatory diagram of a system configuration example of the abnormality detection system 200. In FIG. 2, the abnormality detection system 200 includes a server 201, a wearable terminal 202, and a client apparatus 203. The server 201, the wearable terminal 202, and the client apparatus 203 in the abnormality detection system 200 are connected through a wired or wireless network 210. The network 210 is, for example, the Internet, a mobile communication network, a local area network (LAN), or a wide area network (WAN).

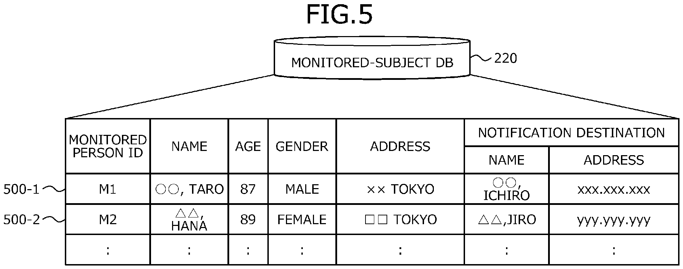

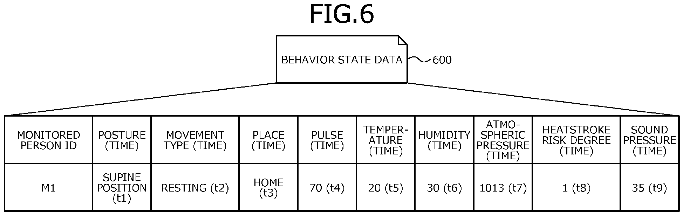

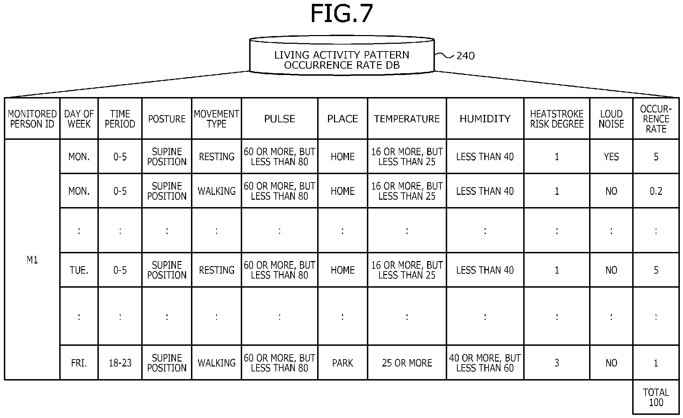

The server 201 is a computer having a monitored-subject database (DB) 220, a behavior state data DB 230, and a living activity pattern occurrence rate DB 240 and detecting an abnormality of a monitored subject. The storage contents of the monitored-subject DB 220 and the living activity pattern occurrence rate DB 240 will be described later with reference to FIGs. 5 and 7. A specific example of behavior state data accumulated in the behavior state data DB 230 will be described later with reference to FIG. 6.

The wearable terminal 202 is a computer attached to a monitored person and is a terminal device of a wristband type, a pendant type, or a badge type, for example. The client apparatus 203 is a computer used by a monitoring person and is a smartphone, a personal computer (PC), or a tablet terminal, for example. The monitoring person is a family member or a caregiver of the monitored person, for example.

Although only the one wearable terminal 202 and the one client apparatus 203 are depicted in FIG. 2, the present invention is not limited hereto. For example, the wearable terminal 202 is provided for each monitored person, and the client apparatus 203 is provided for each monitoring person.

### (Hardware Configuration Example of Server 201)

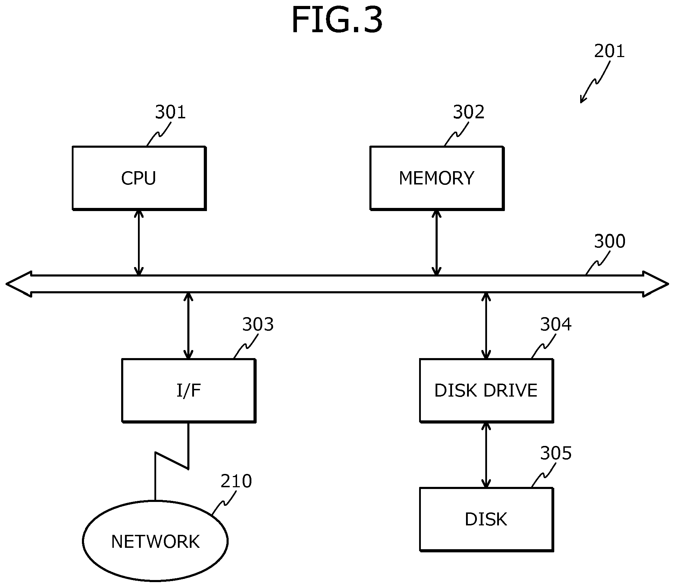

FIG. 3 is a block diagram of a hardware configuration example of a server 201. In FIG. 3, the server 201 has a central processing unit (CPU) 301, a memory 302, an interface (I/F) 303, a disk drive 304, and a disk 305. The constituent units are connected to each other through a bus 300.

The CPU 301 is responsible for the overall control of the server 201. The memory 302 includes, for example, a read-only memory (ROM), a random access memory (RAM), and a flash ROM, etc. In particular, for example, the flash ROM and the ROM store various programs; and the RAM is used as a work area of the CPU 301. Programs stored in the memory 302 are loaded onto the CPU 301 and encoded processes are executed by the CPU 301.

The I/F 303 is connected to a network 210 through a communications line and is connected to an external computer (for example, refer to the wearable terminal 202, the client apparatus 203 depicted in FIG. 2), via the network 210. The I/F 303 administers an internal interface with the network 210, and controls the input and output of data from an external computer. The I/F 303 may be, for example, a modem, a LAN adapter, or the like.

The disk drive 304, under the control of the CPU 301, controls the reading and writing of data with respect to the disk 305. The disk 305 stores data written thereto under the control of the disk drive 304. The disk 305 may be, for example, a magnetic disk, an optical disk, or the like.

In addition to the configuration described above, the server 201 may have, for example a solid state drive (SSD), a keyboard, a mouse, a display, etc. Further, the client apparatus 203 depicted in FIG. 2 may be realized by a hardware configuration similar to the hardware configuration of the server 201.

### (Hardware Configuration Example of Wearable Terminal 202)

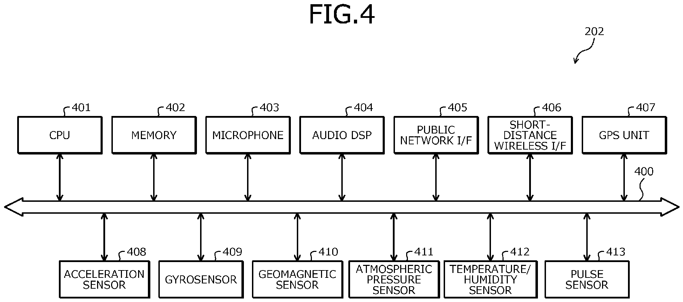

FIG. 4 is a block diagram of a hardware configuration example of the wearable terminal 202. In FIG. 4, the wearable terminal 202 has a CPU 401, a memory 402, a microphone 403, an audio digital signal processor (DSP) 404, a public network I/F 405, a short-distance wireless I/F 406, a Global Positioning System (GPS) unit 407, an acceleration sensor 408, a gyro sensor 409, a geomagnetic sensor 410, an atmospheric pressure sensor 411, a temperature/humidity sensor 412, and a pulse sensor 413. The constituent units are connected to each other through a bus 400.

The CPU 401 is responsible for the overall control of the wearable terminal 202. The memory 402 includes a ROM, a RAM, and a flash ROM, for example. For example, the flash ROM and the ROM store various programs and the RAM is used as a work area of the CPU 401. The programs stored in the memory 402 are loaded onto the CPU 401 and encoded processes are executed by the CPU 401.

The microphone 403 converts sound into an electrical signal. The audio DSP 404 is connected to the microphone 403 and is an arithmetic processing apparatus for executing digital signal processing.

The public network I/F 405 has a wireless communication circuit and an antenna, and is connected to the network 210 through a base station of a mobile communications network, for example, and connected to another computer (e.g., the server 201) via the network 210. The public network I/F 405 is responsible for an internal interface with the network 210 and controls the input and output of data from the other computer.

The short-distance wireless I/F 406 has a wireless communication circuit and an antenna and is connected to a wireless network and connected to another computer via the wireless network. The short-distance wireless I/F 406 is responsible for an internal interface with the wireless network, and controls the input and output of data from the other computer. An example of the short-distance wireless communication is communication using a wireless LAN or Bluetooth (registered trademark), for example.

The GPS unit 407 receives radio waves from GPS satellites and outputs the positional information of the terminal. The positional information of the terminal is, for example, information identifying one point on the earth, such as latitude, longitude, and altitude. The wearable terminal 202 may correct the positional information output from the GPS unit 407 by Differential GPS (DGPS).

The acceleration sensor 408 is a sensor that detects acceleration. The gyro sensor 409 is a sensor that detects angular velocity. The geomagnetic sensor 410 is a sensor that detects the earth's magnetic field along multiple axes. The atmospheric pressure sensor 411 is a sensor that detects altitude. The temperature/humidity sensor 412 is a sensor that detects temperature and humidity. The pulse sensor 413 is a sensor that detects a pulse value.

In addition to the constituent units described above, the wearable terminal 202 may include an input apparatus and a display, for example.

### (Storage Contents of Monitored-Subject DB 220)

The storage contents of the monitored-subject DB 220 included in the server 201 will be described. The monitored-subject DB 220 is implemented by a storage apparatus such as the memory 302 and the disk 305 depicted in FIG. 3, for example.

FIG. 5 is an explanatory diagram of an example of the storage contents of the monitored-subject DB 220. In FIG. 5, the monitored-subject DB 220 has fields of monitored person ID, name, age, gender, address, and notification destination and stores information set in the fields as records of monitored-subject information (e.g., monitored-subject information 500-1, 500-2).

The monitored person ID is an identifier identifying the monitored person. The name is the name of the monitored person. The age is the age of the monitored person. The gender is the sex of the monitored person. The address is the address of the monitored person. The notification destination is the name and address of the notification destination to be notified of an abnormality of the monitored person. For the notification destination, for example, the name and address of a family member or a caregiver defined as the monitoring person are set.

### (Specific Example of Behavior State Data)

A specific example of the behavior state data accumulated in the behavior state data DB 230 included in the server 201 will be described. The behavior state data DB 230 is implemented by a storage apparatus such as the memory 302 and the disk 305 depicted in FIG. 3, for example.

FIG. 6 is an explanatory diagram of a specific example of the behavior state data. In FIG. 6, behavior state data 600 is an example of information indicative of when the monitored person assumes what kind of posture in what state, and is collected by the wearable terminal 202 and uploaded to the server 201.

For example, the behavior state data 600 indicates values of respective items of a posture, a movement type, a place, a pulse rate, a temperature, a humidity, an atmospheric pressure, a heatstroke risk degree, and a sound pressure detected in the wearable terminal 202 in correlation with the monitored person ID. A time (e.g., time t1 to t9) corresponding to each of the items indicates the time when the value of each of the items is detected. However, the values of the items are detected at substantially the same timing, and a time difference between the times is assumed to be negligibly small.

The posture indicates the body posture of the monitored person. The posture is set to any of the standing position, the sitting position, and the supine position, for example. The movement type indicates the movement type when the posture of the monitored person is detected. The movement type is set to, for example, walking, running, resting, riding in a vehicle, or using an elevator or an escalator. The running indicates a state in which the monitored person is running.

The place indicates the place where the posture of the monitored person is detected. For example, the place is set to a landmark such as the monitored person's home, a hospital, and a park. The pulse rate indicates the pulse rate (unit: times/minute) when the posture of the monitored person is detected. The temperature indicates the surrounding temperature (unit: degrees C) when the posture of the monitored person is detected. The humidity indicates the humidity (unit: %) when the posture of the monitored person is detected.

The atmospheric pressure indicates the atmospheric pressure (unit: hPa) when the posture of the monitored person is detected. The heatstroke risk degree indicates the heatstroke risk degree when the posture of the monitored person is detected. The heatstroke risk degree is set to any one of Levels 1 to 4, for example. When the level is higher, the heatstroke risk degree indicates a higher heatstroke risk.

The sound pressure indicates the sound pressure (unit: dB) of the sound when the posture of the monitored person is detected. The sound pressure is set when the measured value is equal to or greater than a predetermined sound pressure (e.g., 30 dB or more). When the measured value is less than the predetermined sound pressure, for example, "- (Null)" is set. The sound pressure is used for judging whether a loud sound has occurred in the surroundings when the posture of the monitored person is detected.

### (Storage Contents of Living Activity Pattern Occurrence Rate DB 240)

The storage contents of the living activity pattern occurrence rate DB 240 included in the server 201 will be described. The living activity pattern occurrence rate DB 240 is implemented by a storage apparatus such as the memory 302 and the disk 305 depicted in FIG. 3, for example.

FIG. 7 is an explanatory diagram of an example of the storage contents of the living activity pattern occurrence rate DB 240. In FIG. 7, the living activity pattern occurrence rate DB 240 stores an occurrence rate indicative of a certainty of the monitored person assuming the predetermined posture for each living activity pattern in correlation with the monitored person ID.

The living activity pattern indicates when and in what state the monitored person assumes the predetermined posture, and is identified by multiple items, for example. In the example of FIG. 7, the multiple items are "day of week", "time period", "posture", "movement type", "pulse rate", "place", "temperature", "humidity", "heatstroke risk degree", and "loud sound".

The "day of week" is set to any of Monday to Sunday. The "time period" is set to any of a time period (0-5) from 0 o'clock to 5 o'clock, a time period (6-11) from 6 o'clock to 11 o'clock, a time period (12-17) from 12 o'clock to 17 o'clock, and a time period (18-23) from 18 o'clock to 23 o'clock.

The "posture" is set to, for example, any of the standing position, the sitting position, and the supine position depending on what kind of abnormality is to be detected of the monitored person. For example, when "falling" of the monitored person is to be detected, the "supine position" is set as depicted in FIG. 7. The "movement type" is set to walking, running, resting, riding in a vehicle, using an elevator or an escalator, etc.

The "pulse rate" is set to less than 60, 60 or more and less than 80, or 80 or more (unit: times/minute). The "place" is set to a landmark such as the home, a hospital, and a park, or indoor and outdoor places, etc. The "temperature" is set to less than 16, 16 or more and less than 25, or 25 or more (unit: degrees C).

The "humidity" is set to less than 40, 40 or more and less than 60, or 60 or more (unit: %). The "heatstroke risk degree" is set to any of Levels 1 to 4. The "loud sound" is set to presence or absence. The presence indicates that a loud sound (e.g., a sound with a sound pressure of 30 dB or more) has occurred. The absence indicates that no loud noise has occurred.

In FIG. 7, a monitored person ID "M1" of a monitored person M1 is depicted as an example. For example, in the case of the day of week "Monday", the time period "0-5", the movement type "stationary", the pulse rate "60 or more and less than 80", the place "home", the temperature "16 or more and less than 25", the humidity "less than 40", the heatstroke risk degree "1", and the large sound "presence", the occurrence rate of the monitored person M1 assuming the posture of "supine position" is "5%".

The occurrence rate of each living behavior pattern indicative of the certainty of the monitored person assuming the posture of "supine position" is normalized such that when all the living behavior patterns are added together, the total is 100%. In the living activity pattern occurrence rate DB 240, the occurrence rate based on typical living activity patterns of older adults may be stored in an initial state.

### (Functional Configuration Example of Wearable Terminal 202)

A functional configuration example of the wearable terminal 202 will be described.

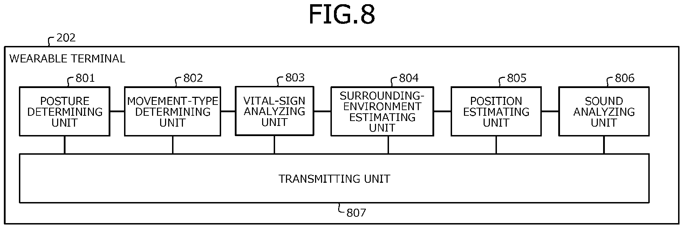

FIG. 8 is a block diagram of a functional configuration example of the wearable terminal 202. In FIG. 8, the wearable terminal 202 includes a posture determining unit 801, a movement-type determining unit 802, a vital-sign analyzing unit 803, a surrounding-environment estimating unit 804, a position estimating unit 805, a sound analyzing unit 806, and a transmitting unit 807. The posture determining unit 801 to the transmitting unit 807 are functions acting as a control unit and, for example, the functions thereof are implemented by causing the CPU 401 to execute a program stored in the memory 402 depicted in FIG. 4, for example, or by the public network I/F 405 and the short-distance wireless I/F 406. The process results of the functional units are stored in the memory 402, for example.

The posture determining unit 801 determines the posture of the monitored person based on the output values of the various sensors 408 to 413 (or the GPS unit 407). For example, the posture determining unit 801 acquires an output value from the atmospheric pressure sensor 411. The posture determining unit 801 then calculates the height (altitude) from the acquired output value of the atmospheric pressure sensor 411 and calculates a change amount from a standing height.

The standing height refers to the height of the monitored person in a standing state. In particular, the standing height indicates, for example, the height (altitude) of the attachment position of the wearable terminal 202 in the standing state of the monitored person. The standing height may manually be set, or the posture determining unit 801 may detect walking of the monitored person from the output value of the acceleration sensor 408, for example, and may set the height acquired from the output value of the atmospheric pressure sensor 411 during the walking as the standing height.

For example, when the calculated change amount from the standing height is less than a first threshold value, the posture determining unit 801 determines that the posture of the monitored person is the "standing position". For example, when the calculated change amount from the standing height is the first threshold value or more and less than a second threshold value, the posture determining unit 801 determines that the posture of the monitored person is the "sitting position". For example, when the calculated change amount from the standing height is the second threshold value or more, the posture determining unit 801 determines that the posture of the monitored person is the "supine position".

In this way, the posture of the monitored person may be detected. The first threshold value and the second threshold value may be set arbitrarily and are set with consideration of the height of the monitored person and the attachment position of the wearable terminal 202, for example. For example, the first threshold value is set to a value of about 30 cm and the second threshold value is set to a value of about 90 cm.

The posture determining unit 801 records a determination result to the memory 402 with time information added thereto. The time information is information indicative of the current date and time, for example, and may be acquired from the OS, etc. For example, the posture determining unit 801 sets the determined posture of the monitored person and the time information in the behavior state data (see, e.g., FIG. 6).

The movement-type determining unit 802 determines the movement type of the monitored person based on the output values from the various sensors 408 to 413 (or the GPS unit 407). For example, the movement-type determining unit 802 acquires the output values of the acceleration sensor 408, the gyro sensor 409, the geomagnetic sensor 410, and the atmospheric pressure sensor 411.

The movement-type determining unit 802 then detects walking, running, or resting of the monitored person from the acquired output values of the various sensors 408 to 411. The movement-type determining unit 802 may detect that the person is riding in a vehicle from the output values of the various sensors 408 to 411. Examples of the vehicles include a car, a bus, a train, etc. The movement-type determining unit 802 may detect that the person is using an elevator or an escalator from the output values of the various sensors 408 to 411.

The movement-type determining unit 802 records a determination result in the memory 402 with time information added thereto. For example, the movement-type determining unit 802 sets the determined movement type of the monitored person and the time information in the behavior state data (see, e.g., FIG. 6).

The vital-sign analyzing unit 803 analyzes the vital signs of the monitored person based on the output values of the temperature/humidity sensor 412 and the pulse sensor 413. Examples of the vital signs include a pulse rate (times/minute), a body temperature (degrees), etc. For example, the vital-sign analyzing unit 803 calculates the pulse rate (times/minute) of the monitored person from the output value of the pulse sensor 413.

The vital-sign analyzing unit 803 records an analysis result to the memory 402 with time information added thereto. For example, the vital-sign analyzing unit 803 sets the analyzed pulse rate (times/minute) of the monitored person and the time information in the behavior state data (see, e.g., FIG. 6).

The surrounding-environment estimating unit 804 estimates the surrounding environment of the monitored person based on the output values of the atmospheric pressure sensor 411 and the temperature/humidity sensor 412. The surrounding environment is identified by at least any of temperature, humidity, atmospheric pressure, and wet-bulb globe temperature around the monitored person, for example. For example, the surrounding-environment estimating unit 804 detects the output value of the atmospheric pressure sensor 411 as the atmospheric pressure around the monitored person.

For example, the surrounding-environment estimating unit 804 detects the output values (temperature, humidity) of the temperature/humidity sensor 412 as the temperature and the humidity around the monitored person. However, the temperature measured by the temperature/humidity sensor 412 may be higher than the actual surrounding temperature due to heat generation of the wearable terminal 202, for example. Therefore, for example, the surrounding-environment estimating unit 804 may subtract a predetermined value from the output value (temperature) of the temperature/humidity sensor 412 to correct the output value (temperature) of the temperature/humidity sensor 412 to the surrounding temperature.

For example, the surrounding-environment estimating unit 804 may calculate the wet-bulb globe temperature from the output value of the temperature/humidity sensor 412 to identify the heatstroke risk degree. The wet-bulb globe temperature (WBGT) is an index obtained from humidity, radiant heat, and atmospheric temperature having a significant influence on a heat balance of a human body and is used for risk assessment under a hot environment etc. (unit: degrees C).

For example, the surrounding-environment estimating unit 804 calculates the wet-bulb globe temperature based on the globe temperature, the wet-bulb temperature, and the dry-bulb temperature. The surrounding-environment estimating unit 804 refers to information indicative of a correspondence relationship between the wet-bulb globe temperature and the heatstroke risk degree to identify the heatstroke risk degree corresponding to the calculated wet-bulb globe temperature.

For example, The heatstroke risk degree is specified to Level 1 when the wet-bulb globe temperature is less than 25 degrees C, and the heatstroke risk degree is specified to Level 2 when the wet-bulb globe temperature is 25 degrees C to 28 degrees C. The heatstroke risk degree is specified to Level 3 when the wet-bulb globe temperature is 28 degrees C to 31 degrees C, and the heatstroke risk degree is specified to Level 4 when the wet-bulb globe temperature is 31 degrees C or higher.

The globe temperature, the wet-bulb temperature, and the dry-bulb temperature may be acquired by accessing an external computer providing weather information, for example. The calculation formula of the wet-bulb globe temperature differs depending on whether the place is indoors or outdoors. Therefore, for example, the surrounding-environment estimating unit 804 may identify whether the place is indoors or outdoors from the output values of the GPS unit 407 etc., to obtain the wet-bulb globe temperature. However, the surrounding-environment estimating unit 804 may obtain the wet-bulb globe temperature on the basis that the person is staying either inside or outside.

The surrounding-environment estimating unit 804 records an estimation result to the memory 402 with time information added thereto. For example, the surrounding-environment estimating unit 804 sets the estimated surrounding environment (e.g., the temperature, the humidity, the atmospheric pressure, the heatstroke risk degree) of the monitored person and the time information in the behavior state data (see, e.g., FIG. 6).

The position estimating unit 805 estimates the current position of the monitored person based on the output values of the GPS unit 407 or the various sensors 408 to 411. For example, the position estimating unit 805 acquires the positional information (e.g., latitude, longitude, and altitude) of the terminal by using the output value of the GPS unit 407, autonomous navigation, etc.

The position estimating unit 805 then refers to the positional information of landmarks registered in advance, to identify a landmark in the vicinity of the point indicated by the acquired positional information of the terminal. If no neighboring landmark may be identified, the position estimating unit 805 may identify at least whether the place is indoors or outdoors.

The position estimating unit 805 may estimate the current position of the terminal by communicating through the short-distance wireless I/F 406 with an access point of a wireless LAN, etc.

The position estimating unit 805 records an estimation result to the memory 402 with time information added thereto. For example, the position estimating unit 805 sets the estimated current position (e.g., the landmark, an indoor or outdoor place) and the time information in the behavior state data (see, e.g., FIG. 6).

The sound analyzing unit 806 analyzes sound information of the sound input to the microphone 403. For example, the sound analyzing unit 806 acquires the sound information of the sound input to the microphone 403. The sound analyzing unit 806 then activates the voice DSP 404 and inputs the acquired sound information to measure the sound pressure. The sound analyzing unit 806 judges if the measured sound pressure is equal to or greater than a predetermined sound pressure. The predetermined sound pressure may be set arbitrarily and is set to a value (e.g., 30 dB) making it possible to judge that a loud sound has occurred around the monitored person when a sound equal to or greater the predetermined sound pressure is generated, for example.

The sound analyzing unit 806 records an analysis result to the memory 402 with time information added thereto. For example, if the measured sound pressure is equal to or greater than the predetermined value, the sound analyzing unit 806 sets the measured sound pressure and the time information in the behavior state data (e.g., see FIG. 6).

The transmitting unit 807 transmits data indicative of the posture of the monitored person and the time of detection of the posture to the server 201. For example, the transmitting unit 807 transmits the determination result determined by the posture determination unit 801 to the server 201 together with the time information added to the determination result.

The transmitting unit 807 transmits data indicative of the movement type of the monitored person and the time of determination of the movement type to the server 201. For example, the transmitting unit 807 transmits the determination result determined by the movement-type determining unit 802 to the server 201 together with the time information added to the determination result.

The transmitting unit 807 transmits data indicative of the vital sign of the monitored person and the time of analysis of the vital sign to the server 201. For example, the transmitting unit 807 transmits the analysis result obtained by the vital-sign analyzing unit 803 to the server 201 together with the time information added to the analysis result.

The transmitting unit 807 transmits data indicative of the surrounding environment of the monitored person and the time of detection of the surrounding environment to the server 201. For example, the transmitting unit 807 transmits the estimation result estimated by the surrounding-environment estimating unit 804 to the server 201 together with the time information added to the estimation result.

The transmitting unit 807 transmits data indicative of the current position of the monitored person and the time of estimation of the current position to the server 201. For example, the transmitting unit 807 transmits the estimation result estimated by the position estimating unit 805 to the server 201 together with the time information added to the estimation result.

The transmitting unit 807 transmits data indicative of the sound pressure of the sound input to the microphone 403 and the time of measurement of the sound pressure to the server 201. For example, the transmitting unit 807 transmits the analysis result obtained by the sound analyzing unit 806 to the server 201 together with the time information added to the analysis result.

For example, the transmitting unit 807 may send the behavior state data 600 as depicted in FIG. 6 to the server 201. Consequently, for example, the various data obtained at substantially the same timing may be uploaded collectively to the server 201.

For example, by using an existing technique, the wearable terminal 202 may estimate whether a falling motion has occurred based on the output values of the various sensors 408 to 411. The wearable terminal 202 may then add an estimation result of whether a falling motion has occurred to the behavior state data for transmission to the server 201, for example.

### (Functional Configuration Example of Server 201)

A functional configuration example of the server 201 will be described.

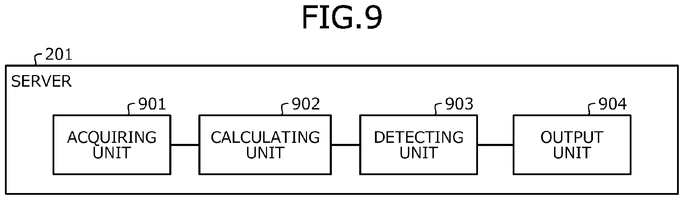

FIG. 9 is a block diagram of a functional configuration example of the server 201. In FIG. 9, the server 201 includes an acquiring unit 901, a calculating unit 902, a detecting unit 903, and an output unit 904. The acquiring unit 901 to the output unit 904 are functions acting as a control unit and, for example, the functions thereof are implemented by causing the CPU 301 to execute a program stored in the storage apparatus such as the memory 302 and the disk 305 depicted in FIG. 3, for example, or by the I/F 303. The process results of the functional units are stored in a storage apparatus such as the memory 302 and the disk 305, for example.

The acquiring unit 901 acquires from the wearable terminal 202, the data indicative of the posture of the monitored person and the time of detection of the posture. The acquiring unit 901 acquires from the wearable terminal 202, the data indicative of the movement type of the monitored person and the time of determination of the movement type.

The acquiring unit 901 acquires from the wearable terminal 202, the data indicative of the vital sign of the monitored person and the time of analysis of the vital sign. The acquiring unit 901 acquires from the wearable terminal 202, the data indicative of the surrounding environment of the monitored person and the time of estimation of the surrounding environment.

The acquiring unit 901 acquires from the wearable terminal 202, the data indicative of the current position of the monitored person and the time of estimation of the current position. The acquiring unit 901 acquires from the wearable terminal 202, the data indicative of the sound pressure of the sound input to the microphone 403 of the wearable terminal 202 and the time of measurement of the sound pressure.

For example, the acquiring unit 901 may acquire the behavior state data (e.g., the behavior state data 600 depicted in FIG. 6) from the wearable terminal 202. Consequently, for example, the various data obtained at substantially the same timing can be acquired collectively from the wearable terminal 202.

The acquired various data are accumulated in the storage apparatus such as the memory 302 and the disk 305. For example, the acquired behavior state data is accumulated in the behavior state data DB 230 (see FIG. 2), for example. If the various data are individually acquired from the wearable terminal 202, for example, the server 201 may accumulate a combination of data in which the times indicated by the respective data are approximately the same time (e.g., having a time difference within one second), as the behavior state data in the behavior state data DB 230.

The calculating unit 902 calculates a certainty of the monitored person assuming the predetermined posture for each of the living activity patterns based on the various data acquired by the acquiring unit 901. The living activity pattern indicates when and in what state the monitored person assumes the predetermined posture.

The predetermined posture is a posture set according to what kind of abnormality is detected from the monitored subject. For example, when the "falling" of the monitored person is detected, the predetermined posture is set to the "supine position", which is a posture when the person performs a motion similar to a falling motion. The certainty of assuming the predetermined posture indicates a degree of certainty that the monitored person assumes the predetermined posture.

For example, the calculating unit 902 may calculate a first certainty by using a Naive Bayes classifier, etc. based on the data indicative of the posture of the monitored person and the time of detection of the posture. The first certainty is the certainty that the monitored person assumes the predetermined posture in each of predetermined time periods.

The predetermined time periods are multiple time periods separated by dividing one day by a certain time interval, for example. For example, if one day is divided by six hours, the predetermined time periods are a time period from 0 o'clock to 5 o'clock, a time period from 6 o'clock to 11 o'clock, a time period from 12 o'clock to 17 o'clock, and a time period from 18 o'clock to 23 o'clock.

A calculation example of the first certainty of the monitored person assuming the posture of "supine position" will be described by taking a case of detecting the "falling" of the monitored person as an example. In this example, the predetermined time periods are defined as a time period T1 from 0 o'clock to 5 o'clock, a time period T2 from 6 o'clock to 11 o'clock, a time period T3 from 12 o'clock to 17 o'clock, and a time period T4 from 18 o'clock to 23 o'clock. For simplicity, it is assumed that either the "standing position" or the "supine position" is detected as the posture of the monitored person.

First, the calculating unit 902 counts numbers C_{R}1 to C_{R}4 and numbers C_{G}1 to C_{G}4 for the respective time periods T1 to T4 based on the behavior state data of each monitored person, accumulated in the behavior state data DB 230, for example. The numbers C_{R}1 to C_{R}4 are the numbers of times the monitored person assumes the posture "standing position" in the respective time periods T1 to T4. The numbers C_{G}1 to C_{G}4 are the numbers of times the monitored person assumes the posture "supine position" in the respective time periods T1 to T4.

For example, if the behavior state data exists that indicates the time "May 11, 2015 at 00:15:23" when the posture "supine position" of the monitored person is detected, the number C_{G}1 of times of the monitored person assuming the posture of "supine position" in the time period T1 is incremented.

For example, it is assumed that, as a result, for all the time periods T1 to T4, the number C_{R} (=C_{R}1+C_{R}2+C_{R}3+C_{R}4) of times of the monitored person assuming the "standing position" is "85" while the number C_{G} (=C_{G}1+C_{G}2+C_{G}3+C_{G}4) of times of the monitored person taking the "supine position" is "63". For example, it is also assumed that the number C_{G}1 of times of the monitored person taking the "supine position" in the time period T1 is "25".

In this case, the calculating unit 902 can multiply the proportion of the number C_{G} to the total number C (=C_{R}+C_{G}=148) by the proportion of the number C_{G}1 to the number C_{G} so as to calculate the probability of assuming the posture of "supine position" during the time period T1. In this example, the probability of assuming the posture of "supine position" in the time period T1 is "0.1689 (≈63/148×25/63)".

Subsequently, for example, the calculating unit 902 normalizes the probability of the monitored person assuming the posture of "supine position" in each of the time periods T1 to T4 so as to calculate the occurrence rate indicative of the first certainty of the monitored person assuming the posture of "supine position" in each of the time periods T1 to T4. For example, the calculating unit 902 performs the normalization such that the sum of the occurrence rates indicative of the first certainty of the monitored person assuming the posture of "supine position" in the time periods T1 to T4 is 100%.

This makes it possible to calculate information (e.g. the occurrence rate) indicative of the first certainty of the monitored person assuming the predetermined posture (e.g., the supine position) in each of the predetermined time periods (e.g., the time periods T1 to T4).

The calculation unit 902 may calculate a second certainty by using a Naive Bayes classifier, etc. based on the data indicative of the posture of the monitored person, the time of detection of the posture, and the place, for example. The second certainty is the certainty that the monitored person assumes the predetermined posture in each of the predetermined time periods at each of predetermined places. The predetermined place is a place where the monitored person may be present, for example, and may be a landmark such as the home, a park, and a hospital, indoor and outdoor places, etc.

A calculation example of the second certainty of the monitored person assuming the posture of "supine position" will be described by taking a case of detecting the "falling" of the monitored person as an example. In this example, the predetermined time periods are defined as the time periods T1 to T4 described above, and the predetermined places are defined as a place P1 indicative of the home, a place P2 indicative of a park, and a place P3 indicative of a hospital. For simplicity, it is assumed that either the "standing position" or the "supine position" is detected as the posture of the monitored person.

First, the calculating unit 902 counts numbers C'_{R}1 to C'_{R}3 and numbers C'_{G}1 to C'_{G}3 for the respective places P1 to P3 based on the behavior state data of each monitored person, for example. The numbers C'_{R}1 to C'_{R}3 are the numbers of times the monitored person assumes the posture "standing position" in the respective places P1 to P3. The numbers C'_{G}1 to C'_{G}3 are the numbers of times the monitored person assumes the posture "supine position" in the respective places P1 to P3.

For example, if the behavior state data exists that indicates the place P1 where the posture "supine position" of the monitored person is detected, the number C'_{G}1 of times of the monitored person assuming the posture of "supine position" in the place P1 is incremented.

For example, it is assumed that, as a result, for all the places P1 to P3, the number C'_{R} (=C'_{R}1+C'_{R}2+C'_{R}3) of times of the monitored person assuming the "standing position" is "85" while the number C'_{G} (=C'_{G}1+C'_{G}2+C'_{G}3) of times of the monitored person assuming the "supine position" is "63". For example, it is also assumed that the number C'_{G}1 of times of the monitored person assuming the "supine position" at the place P1 is "6".

In this case, the calculating unit 902 may multiply the proportion of the number C'_{G} to the total number C (=C'_{R}+C'_{G}=148) by the proportion of the number C'_{G}1 to the number C'_{G} so as to calculate the probability of assuming the posture of "supine position" at the place P1. In this example, the probability of assuming the posture of "supine position" at the place P1 is "0.0405 (≈63/148×6/63)".

The calculating unit 902 then multiplies the calculated probability of assuming the posture of "supine position" at the place P1 and the probability of the monitored person assuming the posture of "supine position" during the time period T1 to calculate a second probability of the monitored person assuming the posture of "supine position" during the time period T1 at the place P1. It is assumed that the probability of the monitored person assuming the posture of "supine position" in the time period T1 is calculated as "0.1689".

In this case, the probability of the monitored person assuming the posture of "supine position" during the time period T1 at the place P1 is "0.00684 (≈00405×0.1689)". For other combinations of the place and the time period, the probability of the monitored person assuming the posture of "supine position" may be obtained in the same way.

For example, the calculating unit 902 then normalizes the probability of the monitored person assuming the posture of "supine position" in each of the time periods T1 to T4 at each of the places P1 to P3 so as to calculate the occurrence rate indicative of the second certainty of the monitored person assuming the posture of "supine position" in each of the time periods T1 to T4 at each of the places P1 to P3.

This makes it possible to calculate information (e.g. the occurrence rate) indicative of the second certainty of the monitored person assuming a predetermined posture (e.g., the supine position) in each of the predetermined time periods (e.g., the time periods T1 to T4) in each of the predetermined places (e.g., the places P1 to P3).

The calculating unit 902 may calculate a third certainty based on the data indicative of the posture of the monitored person, the time of detection of the posture, and the presence/absence of sound equal to or greater than the predetermined sound pressure, for example. The third certainty is the certainty that the monitored person assumes the predetermined posture in each of the predetermined time periods in each of the presence and absence of sound equal to or greater than the predetermined sound pressure. The sound equal to or greater than the predetermined sound pressure is a loud sound startling the monitored person and causing a falling and is, for example, a sound with a sound pressure of 30 dB or more.

For example, the calculating unit 902 calculates the third certainty by using a Naive Bayes classifier, etc. based on the behavior state data of each monitored person accumulated in the behavior state data DB 230. A calculation example of the third certainty is the same as the calculation example of the second certainty described above and therefore, will not be described.

This makes it possible to calculate information (e.g. the occurrence rate) indicative of the third certainty of the monitored person assuming a predetermined posture (e.g., the supine position) in each of the predetermined time periods (e.g., the time periods T1 to T4) in each of the presence and absence of the sound equal to or greater than the predetermined sound pressure.

The calculating unit 902 may calculate a fourth certainty based on the data indicative of the posture of the monitored person, the time of detection of the posture, and the surrounding environment, for example. The fourth certainty is the certainty that the monitored person assumes the predetermined posture in each of the predetermined time periods in each of predetermined surrounding environments. The surrounding environment is identified by at least any of the temperature, the humidity, the atmospheric pressure, and the wet-bulb globe temperature (heatstroke risk degree) around the monitored person, for example.

It is assumed that the surrounding environment is identified by the temperature, the humidity, and the heatstroke risk degree. It is also assumed that the temperature is classified into three categories of "less than 16", "16 or more and less than 25", and "25 or more" (unit: degrees C). It is also assumed that the humidity is classified into three categories of "less than 40", "40 or more and less than 60", and "60 or more" (unit:%). It is also assumed that the heatstroke risk degree is classified into four categories of "Level 1", "Level 2", "Level 3", and "Level 4". In this case, each of the predetermined surrounding environments is identified by a combination of respective categories of the temperature, the humidity, and the heatstroke risk degree.

For example, the calculating unit 902 calculates the fourth certainty by using a Naive Bayes classifier, etc. based on the behavior state data of each monitored person accumulated in the behavior state data DB 230. A calculation example of the fourth certainty is the same as the calculation example of the second certainty described above and therefore, will not be described.

This makes it possible to calculate information (e.g. the occurrence rate) indicative of the fourth certainty of the monitored person assuming a predetermined posture (e.g., the supine position) in each of the predetermined time periods (e.g., the time periods T1 to T4) in each of the predetermined surrounding environments.

The calculating unit 902 may calculate a fifth certainty based on the data indicative of the posture of the monitored person, the time of detection of the posture, and the movement type, for example. The fifth certainty is the certainty that the monitored person assumes the predetermined posture in each of the predetermined time periods in each of predetermined movement type. Examples of the movement type include walking, running, resting, riding in a vehicle (e.g., a car, a bus), using an elevator or an escalator, etc.

For example, the calculating unit 902 calculates the fifth certainty by using a Naive Bayes classifier etc. based on the behavior state data of each monitored person accumulated in the behavior state data DB 230. A calculation example of the fifth certainty is the same as the calculation example of the second certainty described above and therefore, will not be described.

This makes it possible to calculate information (e.g. the occurrence rate) indicative of the fifth certainty of the monitored person assuming a predetermined posture (e.g., the supine position) in each of the predetermined time periods (e.g., the time periods T1 to T4) in each of the predetermined movement type.

The calculating unit 902 may calculate a sixth certainty based on the data indicative of the posture of the monitored person, the time (date and time) of detection of the posture, for example. The sixth certainty is the certainty that the monitored person assumes the predetermined posture in each of the predetermined time periods in each of predetermined day-of-week classifications. The predetermined day-of-week classifications may be set arbitrarily. For example, the day-of-week classifications may be the respective days of the week from Monday to Sunday or may be a "set of Monday to Friday (weekdays)" and a "set of Saturday and Sunday (holidays)", etc.

For example, the calculating unit 902 calculates the sixth certainty by using a Naive Bayes classifier, etc. based on the behavior state data of each monitored person accumulated in the behavior state data DB 230. A calculation example of the sixth certainty is the same as the calculation example of the second certainty described above and therefore, will not be described.

This makes it possible to calculate information (e.g. the occurrence rate) indicative of the sixth certainty of the monitored person assuming a predetermined posture (e.g., the supine position) in each of the predetermined time periods (e.g., the time periods T1 to T4) in each of the predetermined day-of-week classifications.

The calculating unit 902 may calculate a seventh certainty based on the data indicative of the posture of the monitored person, the time of detection of the posture, and the pulse rate, for example. The seventh certainty is the certainty that the monitored person assumes the predetermined posture in each of the predetermined time periods in each of predetermined pulse rate ranges. The predetermined pulse rate range may be set arbitrarily. For example, the predetermined pulse rate ranges are set to "less than 60", "60 or more and less than 80", and "80 or more" (unit: times/minute).

For example, the calculation unit 902 calculates the seventh certainty by using a Naive Bayes classifier, etc. based on the behavior state data of each monitored person accumulated in the behavior state data DB 230. A calculation example of the seventh certainty is the same as the calculation example of the second certainty described above and therefore, will not be described.

This makes it possible to calculate information (e.g. the occurrence rate) indicative of the seventh certainty of the monitored person assuming a predetermined posture (e.g., the supine position) in each of the predetermined time periods (e.g., the time periods T1 to T4) in each of the predetermined pulse rate ranges.

The calculation unit 902 may calculate an eighth certainty that the monitored person assumes the predetermined posture in each of the predetermined time periods with consideration of two or more of items out of "place", "presence/absence of sound equal to or greater than the predetermined sound pressure", "surrounding environment", "movement type", "day-of-week classification", and "pulse rate range".

The occurrence rate depicted in FIG. 7 indicates the eighth certainty of the monitored person assuming the posture of "supine position" in each of the predetermined time periods T1 to T4, calculated with consideration of all the items of "place", "presence/absence of sound equal to or greater than the predetermined sound pressure", "surrounding environment", "movement type", "day-of-week classification", and "pulse rate range".

For example, the occurrence rate "5%" of the monitored person M1 assuming the posture of "supine position" depicted at the top of FIG. 7 may be obtained by multiplying the following probabilities p1 to p9 for normalization. The probabilities p1 to p9 are calculated based on the behavior state data of the monitored person M1 accumulated in the behavior state data DB 230, for example.
p1=the probability of the monitored person M1 assuming the posture of "supine position" on Monday;
p2=the probability of the monitored person M1 assuming the posture of "supine position" in the time period of 0 o'clock to 5 o'clock;
p3=the probability of the monitored person M1 assuming the posture of "supine position" for the movement type "resting";
p4=the probability of the monitored person M1 assuming the posture of "supine position" at a pulse rate (times/minute) of 60 or more and less than 80;
p5=the probability of the monitored person M1 assuming the posture of "supine position" at the place "home";
p6=the probability of the monitored person M1 assuming the posture of "supine position" when a temperature (degrees C) is 16 or more and less than 25;
p7=the probability of the monitored person M1 assuming the posture of "supine position" at a humidity (%) of less than 40;
p8=the probability of the monitored person M1 assuming the posture of "supine position" when the heatstroke risk degree is Level 1; and
p9=the probability of the monitored person M1 assuming the posture of "supine position" in a situation in which a large sound (sound equal to or greater than the predetermined sound pressure) has not occurred.

For example, the calculation unit 902 may recalculate the occurrence rate for each living activity pattern every time the behavior state data is accumulated in the behavior state data DB 230, so as to update the storage contents of the living activity pattern occurrence rate DB 240. The calculating unit 902 may recalculate the occurrence rate for each living activity pattern every predetermined period (e.g., one week) so as to update the storage contents of the living activity pattern occurrence rate DB 240.

The detecting unit 903 refers to the certainty of the monitored person assuming the predetermined posture in each living behavior pattern calculated by the calculating unit 902 to detect an abnormality of the monitored person based on the data acquired by the acquiring unit 901. For example, the detecting unit 903 may refer to the first certainty calculated by the calculating unit 902 to detect an abnormality of the monitored person based on the data indicative of the posture of the monitored person and the time of detection of the posture.

A detection example in the case of detecting the "falling" of the monitored person from the first certainty will be described by taking the behavior state data 600 depicted in FIG. 6 as an example. First, the detecting unit 903 judges whether the posture indicated by the behavior state data 600 is the "supine position". In the example of FIG. 6, it is judged that the posture is the "supine position". The detecting unit 903 then identifies the time period T including time t1 at which the posture "supine position" of the monitored person M1 is detected out of the time periods T1 to T4, for example.

The detecting unit 903 then detects for a falling of the monitored person M1 based on the occurrence rate indicative the first certainty calculated by the calculating unit 902 for the identified time period T. For example, the detecting unit 903 detects a falling of the monitored person M1 if the occurrence rate of the posture "supine position" in the time period T is equal to or less than a preliminarily recorded threshold value Th. The threshold value Th may be set arbitrarily and is set to a value making it possible to judge that the monitored person is highly unlikely to assume the posture of "supine position" if the occurrence rate is equal to or less than the threshold value Th, for example.

As a result, the falling of the monitored person M1 may be detected when the monitored person M1 assumes the posture "supine position" in the time period in which the monitored person M1 is usually highly unlikely to assume the posture of "supine position".

For example, the detecting unit 903 may refer to the second certainty calculated by the calculating unit 902 to detect an abnormality of the monitored person based on the data indicative of the posture of the monitored person, the time of detection of the posture, and the place. A detection example in the case of detecting the "falling" of the monitored person from the second certainty will be described by taking the behavior state data 600 as an example.

First, the detecting unit 903 judges whether the posture indicated by the behavior state data 600 is the "supine position". In the example of FIG. 6, it is judged that the posture is the "supine position". The detecting unit 903 then identifies the time period T including time t1 at which the posture "supine position" of the monitored person M1 is detected, and the place "home", for example.

The detecting unit 903 then detects for a falling of the monitored person M1 based on the occurrence rate indicative the second certainty calculated by the calculating unit 902 for the combination of the identified time period T and the place "home". For example, the detecting unit 903 detects a falling of the monitored person M1 if the occurrence rate of the posture "supine position" in the time period T in the placed "home" is equal to or less than the threshold value Th.

As a result, the falling of the monitored person M1 may be detected when the monitored person M1 assumes the posture "supine position" in the living activity pattern (combination of the time period and the place) in which the monitored person M1 is usually highly unlikely to assume the posture of "supine position".

For example, the detecting unit 903 may refer to the third certainty calculated by the calculating unit 902 to detect an abnormality of the monitored person based on the data indicative of the posture of the monitored person, the time of detection of the posture, and the presence/absence of sound equal to or greater than the predetermined sound pressure. A detection example in the case of detecting the "falling" of the monitored person from the third certainty will be described by taking the behavior state data 600 as an example.

First, the detecting unit 903 judges whether the posture indicated by the behavior state data 600 is the "supine position". In the example of FIG. 6, it is judged that the posture is the "supine position". The detecting unit 903 then identifies the time period T including time t1 at which the posture "supine position" of the monitored person M1 is detected, and the presence/absence of sound equal to or greater than the predetermined sound pressure, for example. In the example of FIG. 6, since the sound pressure "35" is set, it is identified that a sound equal to or greater than the predetermined sound pressure is present.

The detecting unit 903 then detects for a falling of the monitored person M1 based on the occurrence rate indicative the third certainty calculated by the calculating unit 902 for the combination of the identified time period T and the presence of the sound equal to or greater than the predetermined sound pressure. For example, the detecting unit 903 detects a falling of the monitored person M1 if the occurrence rate of the posture "supine position" in the time period T in the presence of the sound equal to or greater than the predetermined sound pressure is equal to or less than the threshold value Th.

As a result, the falling of the monitored person M1 may be detected when the monitored person M1 assumes the posture "supine position" in the living activity pattern (combination of the time period and the loud sound) in which the monitored person M1 is usually highly unlikely to assume the posture of "supine position".

For example, the detecting unit 903 may refer to the fourth certainty calculated by the calculating unit 902 to detect an abnormality of the monitored person based on the data indicative of the posture of the monitored person, the time of detection of the posture, and the surrounding environment. A detection example in the case of detecting the "falling" of the monitored person from the fourth certainty will be described by taking the behavior state data 600 as an example.

First, the detecting unit 903 judges whether the posture indicated by the behavior state data 600 is the "supine position". In the example of FIG. 6, it is judged that the posture is the "supine position". The detecting unit 903 then identifies the time period T including time t1 at which the posture "supine position" of the monitored person M1 is detected, and the surrounding environment (e.g., the temperature, the humidity, the atmospheric pressure, and the heatstroke risk degree).

The detecting unit 903 then detects for a falling of the monitored person M1 based on the occurrence rate indicative the fourth certainty calculated by the calculating unit 902 for the combination of the identified time period T and the surrounding environment. For example, the detecting unit 903 detects a falling of the monitored person M1 if the occurrence rate of the posture "supine position" in the time period T in the surrounding environment is equal to or less than the threshold value Th.

As a result, the falling of the monitored person M1 may be detected when the monitored person M1 assumes the posture "supine position" in the living activity pattern (combination of the time period and the surrounding environment) in which the monitored person M1 is usually highly unlikely to assume the posture of "supine position".

For example, the detecting unit 903 may refer to the fifth certainty calculated by the calculating unit 902 to detect an abnormality of the monitored person based on the data indicative of the posture of the monitored person, the time of detection of the posture, and the movement type. A detection example in the case of detecting the "falling" of the monitored person from the fifth certainty will be described by taking the behavior state data 600 as an example.

First, the detecting unit 903 judges whether the posture indicated by the behavior state data 600 is the "supine position". In the example of FIG. 6, it is judged that the posture is the "supine position". The detecting unit 903 then identifies the time period T including time t1 at which the posture "supine position" of the monitored person M1 is detected, and the movement type. In the example of FIG. 6, the movement type is identified as "resting".

The detecting unit 903 then detects for a falling of the monitored person M1 based on the occurrence rate indicative the fifth certainty calculated by the calculating unit 902 for the combination of the identified time period T and the movement type "resting". For example, the detecting unit 903 detects a falling of the monitored person M1 if the occurrence rate of the posture "supine position" in the time period T at the movement type "resting" is equal to or less than the threshold value Th.

As a result, the falling of the monitored person M1 may be detected when the monitored person M1 assumes the posture "supine position" in the living activity pattern (combination of the time period and the movement type) in which the monitored person M1 is usually highly unlikely to assume the posture of "supine position".

For example, the detecting unit 903 may refer to the sixth certainty calculated by the calculating unit 902 to detect an abnormality of the monitored person based on the data indicative of the posture of the monitored person and the time of detection of the posture. A detection example in the case of detecting the "falling" of the monitored person from the sixth certainty will be described by taking the behavior state data 600 as an example.

First, the detecting unit 903 judges whether the posture indicated by the behavior state data 600 is the "supine position". In the example of FIG. 6, it is judged that the posture is the "supine position". The detecting unit 903 then identifies the time period T including time t1 at which the posture "supine position" of the monitored person M1 is detected, and the day-of-week classification. It is assumed that the day-of-week classification is identified as "Monday".

The detecting unit 903 then detects for a falling of the monitored person M1 based on the occurrence rate indicative the sixth certainty calculated by the calculating unit 902 for the combination of the identified time period T and the day-of-week classification "Monday". For example, the detecting unit 903 detects a falling of the monitored person M1 if the occurrence rate of the posture "supine position" in the time period T in the day-of-week classification "Monday" is equal to or less than the threshold value Th.

As a result, the falling of the monitored person M1 may be detected when the monitored person M1 assumes the posture "supine position" in the living activity pattern (combination of the time period and the day-of-week classification) in which the monitored person M1 is usually highly unlikely to assume the posture of "supine position".

For example, the detecting unit 903 may refer to the seventh certainty calculated by the calculating unit 902 to detect an abnormality of the monitored person based on the data indicative of the posture of the monitored person, the time of detection of the posture, and the pulse rate. A detection example in the case of detecting the "falling" of the monitored person from the seventh certainty will be described by taking the behavior state data 600 as an example.

First, the detecting unit 903 judges whether the posture indicated by the behavior state data 600 is the "supine position". In the example of FIG. 6, it is judged that the posture is the "supine position". The detecting unit 903 then identifies the time period T including time t1 at which the posture "supine position" of the monitored person M1 is detected, and the pulse rate range. It is assumed that the pulse rate range is identified as "60 or more and less than 80" including the pulse rate "70".

The detecting unit 903 then detects for a falling of the monitored person M1 based on the occurrence rate indicative the seventh certainty calculated by the calculating unit 902 for the combination of the identified time period T and the pulse rate range "60 or more and less than 80". For example, the detecting unit 903 detects a falling of the monitored person M1 if the occurrence rate of the posture "supine position" in the time period T in the pulse rate range "60 or more and less than 80" is equal to or less than the threshold value Th.

As a result, the falling of the monitored person M1 may be detected when the monitored person M1 assumes the posture "supine position" in the living activity pattern (combination of the time period and the pulse rate range) in which the monitored person M1 is usually highly unlikely to assume the posture of "supine position".

For example, the detecting unit 903 may refer to the eighth certainty based on the behavior state data. A detection example in the case of detecting the "falling" of the monitored person from the eighth certainty will be described by taking the behavior state data 600 as an example.

First, the detecting unit 903 judges whether the posture indicated by the behavior state data 600 is the "supine position". In the example of FIG. 6, it is judged that the posture is the "supine position". The detecting unit 903 then refers to, for example, the living activity pattern occurrence rate DB 240 to identify the occurrence rate of the living activity pattern similar to the living activity pattern indicated by the behavior state data 600.

In the example of FIG. 6, the living activity pattern indicated by the behavior state data 600 is similar to the living activity pattern depicted at the top of FIG. 7. Therefore, the occurrence rate "5%" of the monitored person M1 assuming the posture of "supine position" is identified from the living activity pattern occurrence rate DB 240. The detection unit 903 then detects a falling of the monitored person M1 if the identified occurrence rate "5%" is equal to or less than the threshold value Th.

As a result, the falling of the monitored person M1 may be detected when the monitored person M1 assumes the posture "supine position" in the living activity pattern (combination of the time period, the place, the presence/absence of the loud sound, the surrounding environment, the movement type, the day-of-week classification, and the pulse rate) in which the monitored person M1 is usually highly unlikely to assume the posture of "supine position".

The detection unit 903 may detect the falling of the monitored person M1, for example, if the identified occurrence rate "5%" is not within the top n in the descending order of the occurrence rates of the respective living activity patterns of the monitored person M1. The n may be set arbitrarily. As a result, the falling of the monitored person M1 may be detected when the identified occurrence rate "5%" is relatively low among the occurrence rates of the respective living activity patterns of the monitored person M1.

When an abnormality of the monitored person is detected by the detecting unit 903, the output section 904 outputs information indicating that an abnormality of the monitored person is detected. Examples of the output format include transmission to an external computer (e.g., the client apparatus 203) by the public network I/F 405, audio output from a speaker not depicted, etc.

For example, when an abnormality of the monitored person is detected, the output unit 904 may transmit abnormality notification information for notification of the abnormality of the monitored person to a notification destination corresponding to the monitored person. For example, it is assumed that a falling of the monitored person M1 is detected. In this case, the output unit 904 refers to the monitored-subject DB 200 depicted in FIG. 5, for example, and identifies the notification destination (name, address) corresponding to the monitored person M1.

The output unit 904 then transmits the abnormality notification information for notification of the abnormality of the monitored person M1 to the address of the identified notification destination. Consequently, for example, the abnormality notification information for notification of the abnormality of the monitored person M1 is displayed on the client apparatus 203 of the monitoring person that is the notification destination. A specific example of the abnormality notification information will be described.

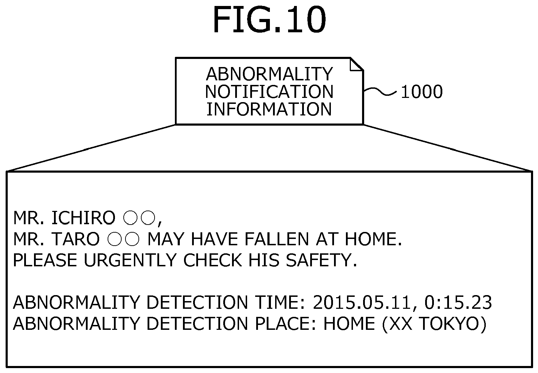

FIG. 10 is an explanatory diagram of a specific example of the abnormality notification information. In FIG. 10, abnormality notification information 1000 is information for notification of the abnormality of the monitored person M1. According to the abnormality notification information 1000, a monitoring person (name: Ichiro ○○) may know that the monitored person M1 (name: Taro ○○) has possibly fallen down at home and may confirm safety, etc.

### (Upload Process Procedure of Wearable Terminal 202)

An upload process procedure of the wearable terminal 202 will be described.

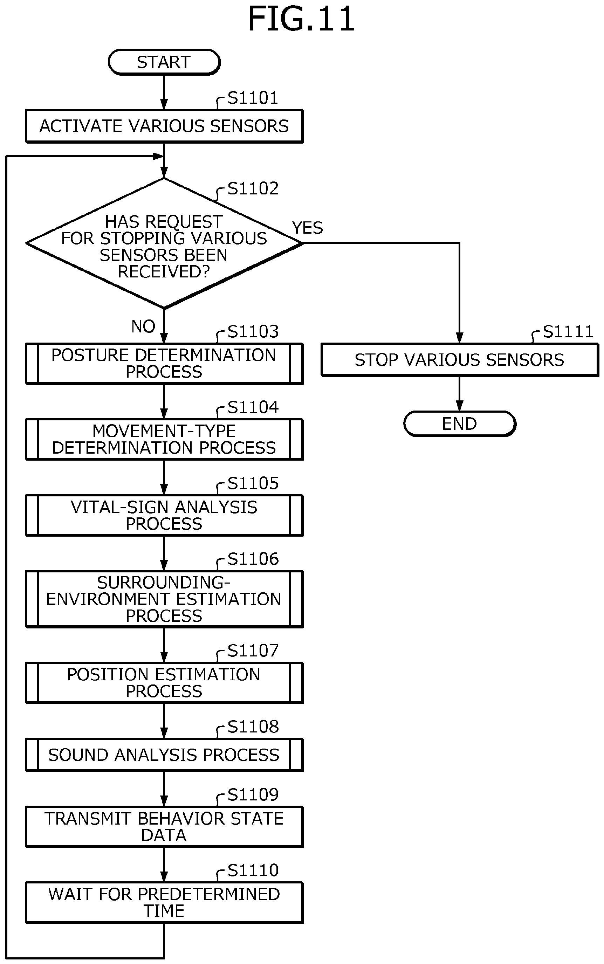

FIG. 11 is a flowchart of an example of the upload process procedure of the wearable terminal 202. In the flowchart of FIG. 11, first, the wearable terminal 202 activates the various sensors 408 to 413 (step S1101).

The wearable terminal 202 then judges whether a request for stopping the various sensors 408 to 413 has been received (step S1102). The request for stopping the various sensors 408 to 413 is made by a user operation input via an input apparatus (not depicted) of the wearable terminal 202, for example.

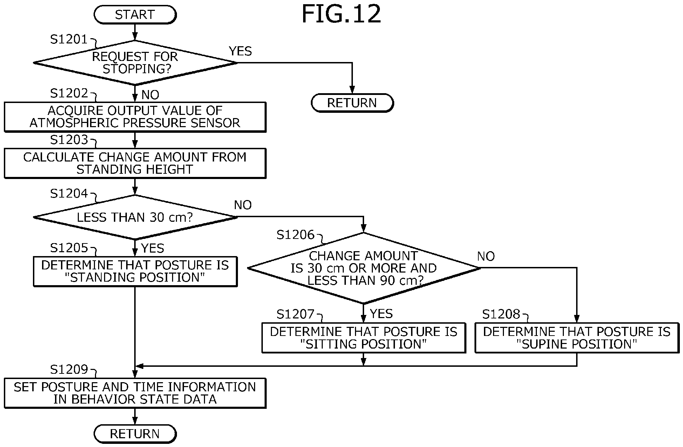

If the request for stopping the various sensors 408 to 413 has not been received (step S1102: NO), the wearable terminal 202 executes a posture determination process of determining the posture of the monitored person (step S1103). A specific process procedure of the posture determination process will be described later with reference to FIG. 12.

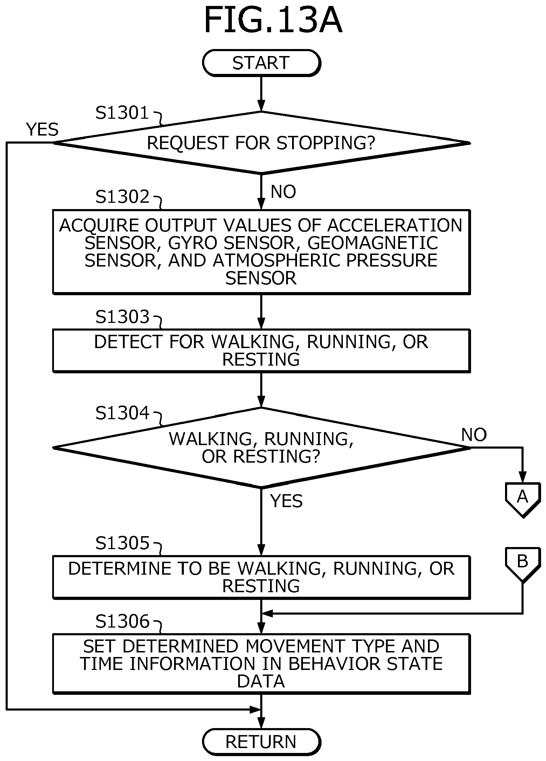

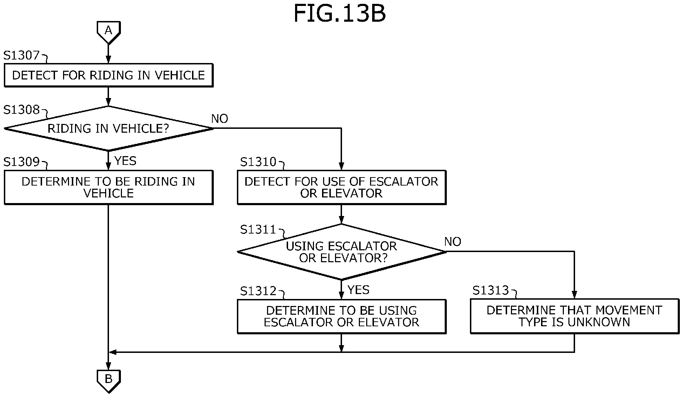

The wearable terminal 202 then executes a movement-type determination process of determining the movement type of the monitored person (step S1104). A specific process procedure of the movement-type determination process will be described later with reference to FIGs. 13A and 13B.

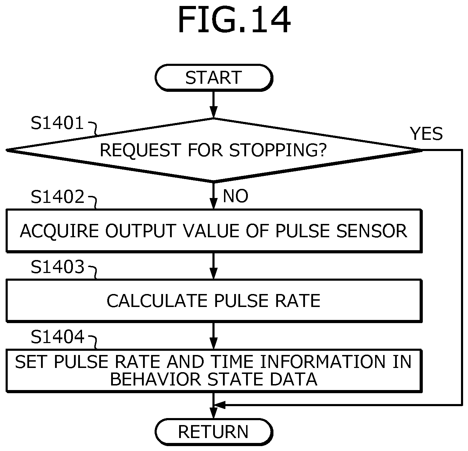

The wearable terminal 202 then executes a vital-sign analysis process of analyzing a vital sign of the monitored person (step S1105). A specific process procedure of the vital-sign analysis process will be described later with reference to FIG. 14.

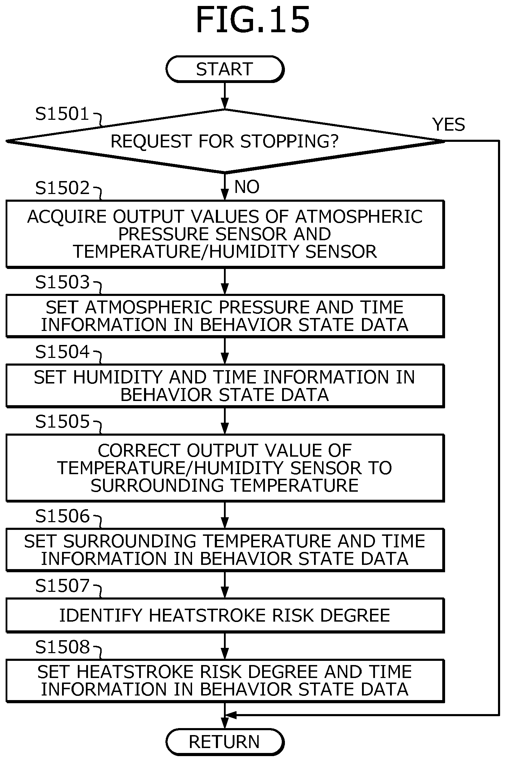

The wearable terminal 202 then executes a surrounding-environment estimation process of estimating the surrounding environment of the monitored person (step S1106). A specific process procedure of the surrounding-environment estimation process will be described later with reference to FIG. 15.

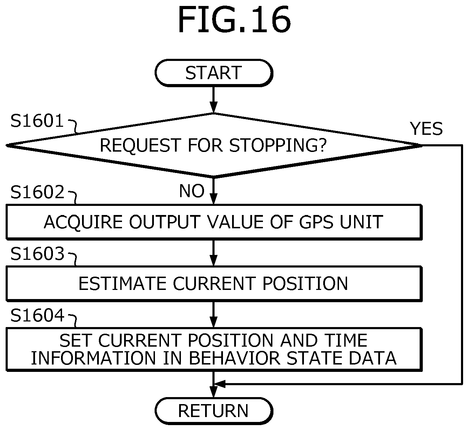

The wearable terminal 202 then executes a position estimation process of estimating the current position of the monitored person (step S1107). A specific process procedure of the position estimation process will be described later with reference to FIG. 16.

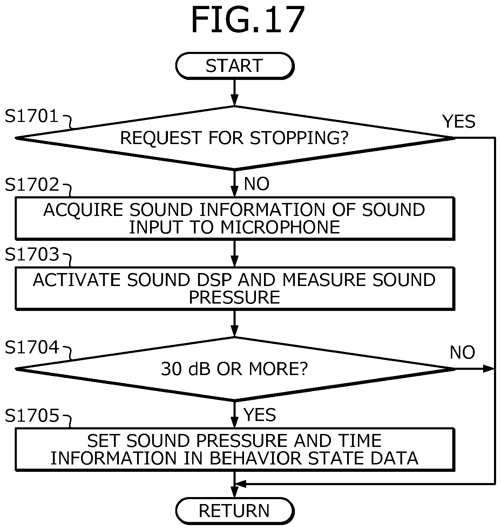

The wearable terminal 202 then executes a sound analysis process of analyzing the sound information of the sound input to the microphone 403 (step S1108). A specific process procedure of the sound analysis process will be described later with reference to FIG. 17.

The wearable terminal 202 transmits the behavior state data to the server 201 (step S1109). The wearable terminal 202 then waits for a predetermined time (step S1110) and returns to step S1102. This waiting time may be set arbitrarily and is set to a time of about 1 to 10 minutes, for example.

If the request for stopping the various sensors 408 to 413 has been received at step S1102 (step S1102: YES), the wearable terminal 202 stops the various sensors 408 to 413 (step S1111) and terminates a series of the processes of this flowchart.

This makes it possible to periodically upload to the server 201, the behavior state data indicative of when the monitored person assumes what kind of posture in what state.

### <Posture Determination Process Procedure>

A specific process procedure of the posture determination process at step S1103 depicted in FIG. 11 will be described with reference to FIG. 12.

FIG. 12 is a flowchart of an example of a specific process procedure of the posture determination process. In the flowchart of FIG. 12, first, the wearable terminal 202 judges whether a request for stopping the posture determination process is made (step S1201). The request for stopping the posture determination process is set by a user operation input via the input apparatus (not depicted) of the wearable terminal 202, for example.

If a request for stopping the posture determination process is not made (step S1201: NO), the wearable terminal 202 acquires the output value of the atmospheric pressure sensor 411 (step S1202). The wearable terminal 202 then obtains the height (altitude) from the acquired output value of the atmospheric pressure sensor 411 and calculates a change amount from the standing height (step S1203).

The wearable terminal 202 judges whether the calculated change amount from the standing height is less than 30 cm (step S1204). If the change amount from the standing height is less than 30 cm (step S1204: YES), the wearable terminal 202 determines that the posture of the monitored person is the "standing position" (step S1205) and goes to step S1209.

On the other hand, if the change amount from the standing height is not less than 30 cm (step S1204: NO), the wearable terminal 202 judges whether the change amount from the standing height is 30 cm or more and less than 90 cm (step S1206). If the change amount from the standing height is 30 cm or more and less than 90 cm (step S1206: YES), the wearable terminal 202 determines that the posture of the monitored person is the "sitting position" (step S1207) and goes to step S1209.

On the other hand, if the change amount from the standing height is not equal to or more than 30 cm and less than 90 cm (step S1206: NO), the wearable terminal 202 determines that the posture of the monitored person is the "supine position" (step S1208). The wearable terminal 202 sets the determined posture and the time information in the behavior state data (step S1209) and returns to the step at which the posture determination process was called. As a result, the posture of the monitored person may be detected.

If a request for stopping the posture determination process is made at step S1201 (step S1201: YES), the wearable terminal 202 returns to the step at which the posture determination process was called. As a result, if it is not necessary to detect the posture of the monitored person, the posture determination process may be stopped.

### <Movement-Means Determination Process Procedure>

A specific process procedure of the movement-type determination process at step S1104 depicted in FIG. 11 will be described with reference to FIGs. 13A and 13B.

FIGs. 13A and 13B are flowcharts of an example of a specific process procedure of the movement-type determination process. In the flowchart of FIG. 13A, first, the wearable terminal 202 judges whether a request for stopping the movement-type determination process is made (step S1301). The request for stopping the movement-type determination process is set by a user operation input via the input apparatus (not depicted) of the wearable terminal 202, for example.

If a request for stopping the movement-type determination process is not made (step S1301: NO), the wearable terminal 202 acquires the output values of the acceleration sensor 408, the gyro sensor 409, the geomagnetic sensor 410, and the atmospheric pressure sensor 411 (step S1302). Subsequently, from the acquired output values of the various sensors 408 to 411, the wearable terminal 202 detects for walking, running, or resting of the monitored person (step S1303).

The wearable terminal 202 then determines whether walking, running, or resting of the monitored person is detected (step S1304). If walking, running, or resting of the monitored person is detected (step S1304: YES), the wearable terminal 202 determines walking, running, or resting as the movement type of the monitored person (step S1305).

The wearable terminal 202 sets the determined movement type and the time information in the behavior state data (step S1306) and returns to the step at which the movement-type determination process was called.

If a request for stopping the movement-type determination process is made in step S1301 (step S1301: YES), the wearable terminal 202 returns to the step at which the movement-type determination process was called. As a result, if it is not necessary to detect the movement type of the monitored person, the movement-type determination process may be stopped.

If walking, running, or resting of the monitored person is not detected at step S1304 (step S1304: NO), the wearable terminal 202 goes to step S1307 depicted in FIG. 13B.

In the flowchart of FIG. 13B, first, the wearable terminal 202 detects for riding in a vehicle, from the output values of the various sensors 408 to 411 (step S1307). The wearable terminal 202 then determines whether riding in a vehicle is detected (step S1308).

If riding in a vehicle is detected (step S1308: YES), the wearable terminal 202 determines riding in a vehicle as the movement type of the monitored person (step S1309) and goes to step S1306 depicted in FIG. 13A.

On the other hand, if riding in a vehicle is not detected (step S1308: NO), the wearable terminal 202 detects for use of an escalator or an elevator, from the output values of the various sensors 408 to 411 (step S1310). The wearable terminal 202 judges whether use an escalator or an elevator is detected (step S1311).

If use an escalator or an elevator is detected (step S1311: YES), the wearable terminal 202 determines use an escalator or an elevator as the movement type of the monitored person (step S1312) and goes to step S1306 depicted in FIG. 13A.

On the other hand, if use an escalator or an elevator is not detected (step S1311: NO), the wearable terminal 202 determines that the movement type of the monitored person is unknown (step S1313) and goes to step S1306 depicted in FIG. 13A. In this manner, the movement type of the monitored person may be detected.

### <Vital-Sign Analysis Process Procedure>

A specific process procedure of the vital-sign analysis process at step S1105 depicted in FIG. 11 will be described with reference to FIG. 14.

FIG. 14 is a flowchart of an example of a specific process procedure of the vital-sign analysis process. In the flowchart of FIG. 14, first, the wearable terminal 202 judges whether a request for stopping the vital-sign analysis process is made (step S1401). The request for stopping the vital-sign analysis process is set by a user operation input via the input apparatus (not depicted) of the wearable terminal 202, for example.

If a request for stopping the vital-sign analysis process is not made (step S1401: NO), the wearable terminal 202 acquires the output value of the pulse sensor 413 (step S1402). The wearable terminal 202 calculates the pulse rate of the monitored person from the acquired output value of the pulse sensor 413 (step S1403).

The wearable terminal 202 then sets the calculated pulse rate and the time information in the behavior state data (step S1404) and returns to the step at which the vital-sign analysis process was called. As a result, the pulse rate (times/minute) of the monitored person may be detected.

If a request for stopping the vital sign analysis is made at step S1401 (step S1401: YES), the wearable terminal 202 returns to the step at which the vital-sign analysis process was called. As a result, if it is not necessary to detect the pulse rate of the monitored person, the vital-sign analysis process may be stopped.

### <Surrounding-Environment Estimation Process Procedure>

A specific process procedure of the surrounding-environment estimation process at step S1106 depicted in FIG. 11 will be described with reference to FIG. 15.

FIG. 15 is a flowchart of an example of a specific process procedure of the surrounding-environment estimation process. In the flowchart of FIG. 15, first, the wearable terminal 202 judges whether a request for stopping the surrounding-environment estimation process is made (step S1501). The request for stopping the surrounding-environment estimation process is set by a user operation input via the input apparatus (not depicted) of the wearable terminal 202, for example.

If a request for stopping the surrounding-environment estimation process is not made (step S1501: NO), the wearable terminal 202 acquires the output values of the atmospheric pressure sensor 411 and the temperature/humidity sensor 412 (step S1502). The wearable terminal 202 then sets the output value (atmospheric pressure) of the atmospheric pressure sensor 411 and the time information in the behavior state data (step S1503). The wearable terminal 202 then sets the output value (humidity) of the temperature/humidity sensor 412 and the time information in the behavior state data (step S1504).

The wearable terminal 202 then corrects the output value (temperature) of the temperature/humidity sensor 412 to a surrounding temperature (step S1505). The wearable terminal 202 sets the corrected surrounding temperature and the time information in the behavior state data (step S1506).

The wearable terminal 202 then identifies the heatstroke risk degree by calculating the wet-bulb globe temperature from the output value of the temperature/humidity sensor 412 (step S1507). The wearable terminal 202 sets the identified heatstroke risk degree and the time information in the behavior state data (step S1508) and returns to the step at which the surrounding-environment estimation process was called. As a result, the surrounding environment of the monitored person may be detected.

If a request for stopping the surrounding-environment estimation process is made at step S1501 (step S1501: YES), the wearable terminal 202 returns to the step at which the surrounding-environment estimation process was called. As a result, if it is not necessary to detect the surrounding environment of the monitored person, the surrounding-environment estimation process may be stopped.

### <Position Estimation Process Procedure>

A specific process procedure of the position estimation process at step S1107 depicted in FIG. 11 will be described with reference to FIG. 16.

FIG. 16 is a flowchart of an example of a specific process procedure of the position estimation process. In the flowchart of FIG. 16, first, the wearable terminal 202 judges whether a request for stopping the position estimation process is made (step S1601). The request for stopping the position estimation process is set by a user operation input via the input apparatus (not depicted) of the wearable terminal 202, for example.

If a request for stopping the position estimation process is not made (step S1601: NO), the wearable terminal 202 acquires the output value of the GPS unit 407 (step S1602). The wearable terminal 202 then estimates the current position of the monitored person from the acquired output value of the GPS unit 407 (step S1603).

The wearable terminal 202 sets the estimated current position of the monitored person and the time information in the behavior state data (step S1604) and returns to the step at which the position estimation process was called. As a result, the current position of the monitored person may be detected.

If a request for stopping the position estimation process is made at step S1601 (step S1601: YES), the wearable terminal 202 returns to the step at which the position estimation process was called. As a result, if it is not necessary to detect the current position of the monitored person, the position estimation process may be stopped.

### <Sound Analysis Process Procedure>

A specific process procedure of the sound analysis process at step S1108 depicted in FIG. 11 will be described with reference to FIG. 17.

FIG. 17 is a flowchart of an example of a specific process procedure of the sound analysis process. In the flowchart of FIG. 17, first, the wearable terminal 202 judges whether a request for stopping the sound analysis process is made (step S1701). The request for stopping the sound analysis process is set by a user operation input via the input apparatus (not depicted) of the wearable terminal 202, for example.

If a request for stopping the sound analysis process is not made (step S1701: NO), the wearable terminal 202 acquires the sound information of the sound input to the microphone 403 (step S1702). The wearable terminal 202 then activates the sound DSP 404 and inputs the acquired sound information to measure the sound pressure (step S1703).

The wearable terminal 202 judges if the measured sound pressure is equal to or more than 30 dB (step S1704). If the measured sound pressure is less than 30 dB (step S1704: NO), the wearable terminal 202 returns to the step at which the sound analysis process was called.

On the other hand, if the measured sound pressure is equal to or greater than 30 dB (step S1704: YES), the wearable terminal 202 sets the measured sound pressure and the time information in the behavior state data (step S1705) and returns to the step at which the sound analysis process was called. As a result, a loud sounds having occurred around the monitored person may be detected.

If a request for stopping the sound analysis process is made at step S1701 (step S1701: YES), the wearable terminal 202 returns to the step at which the sound analysis process was called. As a result, if it is not necessary to detect a sound around the monitored person, the sound analysis process may be stopped.

### (Abnormality Detection Process Procedure of Server 201)

An abnormality detection process procedure of the server 201 will be described.

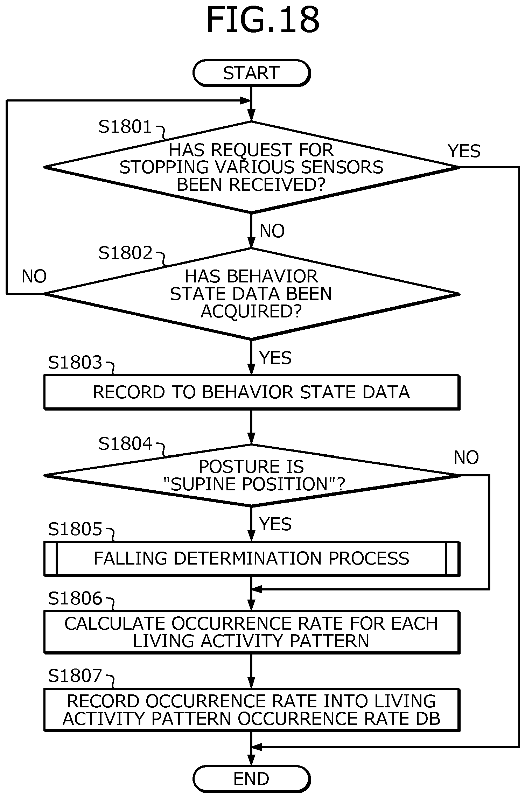

FIG. 18 is a flowchart of an example of the abnormality detection process procedure of the server 201. In the flowchart of FIG. 18, first, the server 201 judges whether a request for stopping an abnormality detection process has been received (step S1801). The request for stopping an abnormality detection process is input from an external computer, for example.

If a request for stopping an abnormality detection process has not been received (step S1801: NO), the server 201 judges whether the behavior state data has been acquired from the wearable terminal 202 (step S1802). If the behavior state data has not been acquired (step S1802: NO), the server 201 returns to step S1801.

On the other hand, if the behavior state data has been acquired (step S1802: YES), the server 201 records the acquired behavior state data in the behavior state data DB 230 (step S1803). The server 201 then determines whether the posture indicated by the acquired behavior state data is the "supine position" (step S1804).

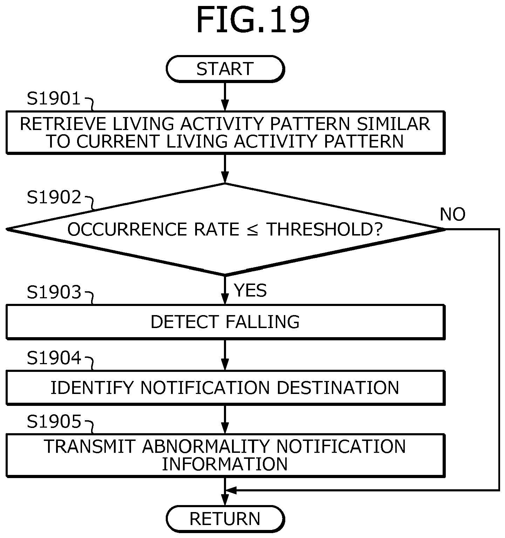

If the posture indicated by the behavior state data is not the "supine position" (step S1804: NO), the server 201 goes to step S1806. On the other hand, if the posture indicated by the behavior state data is the "supine position" (step S1804: YES), the server 201 executes a falling determination process (step S1805). A specific process procedure of the falling determination process will be described later with reference to FIG. 19.

The server 201 calculates an occurrence rate indicative of a certainty that the monitored person assumes the posture "supine position" for each of the living activity patterns based on the behavior state data accumulated in the behavior state data DB 230 (step S1806).

The server 201 records the calculated occurrence rate in each of the living activity patterns into the living activity pattern occurrence rate DB 240 (step S1807) and terminates a series of the processes of the flowchart. As a result, the storage contents of the living activity pattern occurrence rate DB 240 may be updated according to the lifestyle of the monitored person.

If a request for stopping an abnormality detection process has been received at step S1801 (step S1801: YES), the server 201 terminates a series of the processes of the flowchart. As a result, the abnormality detection process by the server 210 may be stopped at an arbitrary timing.

### <Falling Determination Process Procedure>

A specific process procedure of the falling determination process at step S1805 depicted in FIG. 18 will be described with reference to FIG. 19.

FIG. 19 is a flowchart of an example of a specific process procedure of the falling determination process. In the flowchart of FIG. 19, first, the server 201 refers to the living activity pattern occurrence rate DB 240 to retrieve a living activity pattern similar to the living activity pattern indicated by the behavior state data acquired at step S1802 depicted in FIG. 18 (step S1901).

The server 201 then refers to the living activity pattern occurrence rate DB 240 to judge if the occurrence rate of the retrieved living activity pattern is equal to or less than the threshold value Th (step S1902). If the occurrence rate of the living activity pattern is greater than the threshold value Th (step S1902: NO), the server 201 returns to the step at which the falling determination process was called.

On the other hand, if the occurrence rate of the living activity pattern is equal to or less than the threshold value Th (step S1902: YES), the falling of the monitored person is detected (step S1903). The server 201 then refers to the monitored-subject DB 220 and identifies the notification destination corresponding to the monitored person M1 (step S1904).

The server 201 transmits the abnormality notification information for notification of the abnormality of the monitored person to the identified notification destination (step S1905) and returns to the step at which the falling determination process was called. As a result, the monitoring person may be notified of the detection of the falling of the monitored person.

As described above, according to the server 201 of the embodiment, the behavior state data may be acquired from the wearable terminal 202. This makes it possible to identify the time, the movement type, the place, the vital sign, the surrounding environment, and the presence/absence of sound equal to or greater than the predetermined sound pressure when the posture of the monitored person is detected.

According to the server 201, the acquired behavior state data may be accumulated in the behavior state data DB 230 so as to calculate the certainty of the monitored person assuming the predetermined posture for each of the living behavior patterns based on the accumulated behavior state data.

For example, the server 201 may calculate for each of the predetermined time periods, the first certainty that the monitored person assumes the posture "supine position". This makes it possible to judge the certainty that the monitored person assumes the posture "supine position" in each of the predetermined time periods.

For example, the server 201 may calculate for each of the predetermined time periods in each of the predetermined places, a second certainty that the monitored person assumes the predetermined posture. This makes it possible to judge the certainty that the monitored person takes a posture of the posture of "supine position" in each of the predetermined time periods in each of the predetermined places.

For example, the server 201 may calculate for each of the predetermined time periods in each of the presence and absence of sound equal to or greater than the predetermined sound pressure, the third certainty that the monitored person assumes the posture "supine position". This makes it possible to obtain the information indicative of the certainty that the monitored person assumes the posture of "supine position" in each of the predetermined time periods, with consideration of a tendency to fall varying depending on the presence/absence of a loud sound that a person is startled and more likely to fall down when a loud sound has occurred in the surroundings.

For example, the server 201 may calculate for each of the predetermined time periods in each of the predetermined surrounding environments, a fourth certainty that the monitored person assumes the posture "supine position". This makes it possible to obtain the information indicative of the certainty that the monitored person assumes the posture of "supine position" in each of the predetermined time periods, with consideration of a tendency to fall varying depending on the surrounding environment that a person may suffer heatstroke and fall down when the heatstroke risk degree is high, for example.

For example, the server 201 may calculate for each of the predetermined time periods in each of the predetermined movement type, a fifth certainty that the monitored person assumes the posture "supine position". This makes it possible to obtain the information indicative of the certainty in each of the predetermined time periods that the monitored person assumes the posture "supine position", with consideration of a tendency to fall varying depending on the movement type that a person more easily falls down during walking as compared to during resting, for example.

For example, the server 201 may calculate for each of the predetermined time periods in each of the predetermined day-of-week classifications, the sixth certainty that the monitored person assumes the posture "supine position". This makes it possible to obtain the information indicative of the certainty in each of the predetermined time periods in each of the predetermined day-of-week classifications that the monitored person assumes the posture of "supine position".

For example, the server 201 may calculate for each of the predetermined time periods in each of the predetermined pulse rate ranges, a seventh certainty that the monitored person assumes the posture "supine position". This makes it possible to obtain the information indicative of the certainty in each of the predetermined time periods that the monitored person assumes the posture of "supine position", with consideration of a tendency to fall varying depending on the pulse rate that the monitored person more easily falls down because of a poor health condition when the pulse rate is significantly high or low, for example.

According to the server 201, an abnormality of the monitored person may be detected based on the acquired behavior state data by reference to the calculated certainty of the monitored person assuming the predetermined posture for each of the living behavior patterns. This makes it possible to prevent false detection of an abnormality of the monitored person by not detecting an abnormality when it may be judged that a motion is habitually performed by the monitored person even if a motion similar to that at the time of abnormality such as falling is detected.

For example, the server 201 may detect the "falling" of the monitored person based on the occurrence rate indicative of the calculated first certainty for the time period including the time of detection of the posture of "supine position" of the monitored person. This makes it possible to detect the "falling" of the monitored person when the monitored person assumes the posture "supine position" during a time period in which the monitored person is usually highly unlikely to assume the posture of "supine position", so that the monitored person lying down for sleep, etc. may be prevented from being falsely detected as the "falling".

For example, the server 201 may detect the "falling" of the monitored person based on the occurrence rate indicative of the second certainty calculated for the combination of the time period including the time of detection of the posture of "supine position" of the monitored person and the place. This makes it possible to detect the "falling" of the monitored person when the monitored person assumes the posture "supine position" in a living activity pattern (combination of the time period and the place) considered as a pattern in which the monitored person is usually highly unlikely to assume the posture of "supine position", so that the abnormality detection accuracy may be improved.

For example, the server 201 may detect the "falling" of the monitored person based on the occurrence rate indicative of the third certainty calculated for the combination of the time period including the time of detection of the posture of "supine position" of the monitored person and the presence/absence of sound equal to or greater than the predetermined sound pressure. This makes it possible to detect the "falling" of the monitored person when the monitored person assumes the posture "supine position" in a living activity pattern (combination of the time period and the loud sound) considered as a pattern in which the monitored person is usually highly unlikely to assume the posture of "supine position", so that the abnormality detection accuracy may be improved.

For example, the server 201 may detect the "falling" of the monitored person based on the occurrence rate indicative of the fourth certainty calculated for the combination of the time period including the time of detection of the posture of "supine position" of the monitored person and the surrounding environment. This makes it possible to detect the "falling" of the monitored person when the monitored person assumes the posture "supine position" in a living activity pattern (combination of the time period and the surrounding environment) considered as a pattern in which the monitored person is usually highly unlikely to assume the posture of "supine position", so that the abnormality detection accuracy may be improved.

For example, the server 201 may detect the "falling" of the monitored person based on the occurrence rate indicative of the fifth certainty calculated for the combination of the time period including the time of detection of the posture of "supine position" of the monitored person and the movement type. This makes it possible to detect the "falling" of the monitored person when the monitored person assumes the posture "supine position" in a living activity pattern (combination of the time period and the movement type) considered as a pattern in which the monitored person is usually highly unlikely to assume the posture of "supine position", so that the abnormality detection accuracy may be improved.

For example, the server 201 may detect the "falling" of the monitored person based on the occurrence rate indicative of the sixth certainty calculated for the combination of the time period including the time of detection of the posture of "supine position" of the monitored person and the day-of-week classification. This makes it possible to detect the "falling" of the monitored person when the monitored person assumes the posture "supine position" in a living activity pattern (combination of the time period and the day-of-week classification) considered as a pattern in which the monitored person is usually highly unlikely to assume the posture of "supine position", so that the abnormality detection accuracy may be improved.

For example, the server 201 may detect the "falling" of the monitored person based on the occurrence rate indicative of the seventh certainty calculated for the combination of the time period including the time of detection of the posture of "supine position" of the monitored person and the pulse rate range. This makes it possible to detect the "falling" of the monitored person when the monitored person assumes the posture "supine position" in a living activity pattern (combination of the time period and the pulse rate range) considered as a pattern in which the monitored person is usually highly unlikely to assume the posture of "supine position", so that the abnormality detection accuracy may be improved.

According to the server 201, a notification of the abnormality of the monitored person may be made to a notification destination corresponding to the monitored person in response to the detection of the abnormality of the monitored person. Therefore, when the abnormality of the monitored person is detected, a monitoring person such as a family member may be urged to promptly confirm the safety, etc. of the monitored person. Additionally, by preventing the false detection of abnormality of the monitored person, excessive alarms to the monitoring person may be suppressed to reduce the burden of the monitoring person.

The abnormality detection method explained in the present embodiment may be implemented by a computer, such as a personal computer and a workstation, executing a program that is prepared in advance. The program is recorded on a computer-readable recording medium such as a hard disk, a flexible disk, a CD-ROM, an MO, and a DVD, and is executed by being read out from the recording medium by a computer. The program may be distributed through a network such as the Internet.

### EXPLANATIONS OF LETTERS OR NUMERALS

100 information processing apparatus
110 storage unit
200 abnormality detection system
201 server
202 wearable terminal
203 client apparatus
220 monitored-subject DB
230 behavior state data DB
240 living activity pattern occurrence rate DB
600 behavior state data
801 posture determining unit
802 movement-type determining unit
803 vital-sign analyzing unit
804 surrounding-environment estimating unit
805 position estimating unit
806 sound analyzing unit
807 transmitting unit
901 acquiring unit
902 calculating unit
903 detecting unit
904 output unit
1000 abnormality notification information

## Claims

1. An abnormality detection method, comprising:
acquiring, by a computer, data indicating a time when a monitored subject is detected to have assumed a predetermined posture, based on an output value from a sensor corresponding to the monitored subject; and
referencing, by the computer, a storage unit storing information identifying a time period when the monitored subject assumes the predetermined posture and detecting an abnormality of the monitored subject when the time indicated by the acquired data is not included in the time period.

2. The abnormality detection method according to claim 1, further comprising
giving, by the computer, notification of an abnormality of the monitored subject to a notification destination corresponding to the monitored subject, in response to detecting the abnormality of the monitored subject.

3. The abnormality detection method according to claim 2, wherein
the storage unit stores information indicating a certainty of the monitored subject assuming the predetermined posture in each of predetermined time periods, and
the detecting includes referring to the storage unit to detect an abnormality of the monitored subject, based on the certainty of the monitored subject assuming the predetermined posture during the time period including the time indicated by the data.

4. The abnormality detection method according to claim 3, wherein
the storage unit stores information indicating a certainty of the monitored subject assuming the predetermined posture in each of the predetermined time periods in each of predetermined places,
the acquiring includes acquiring data indicating the time and a place when the monitored subject is detected to have assumed the predetermined posture based on the output value from the sensor, and
the detecting includes referring to the storage unit to detect an abnormality of the monitored subject based on the certainty of the monitored subject assuming the predetermined posture in the place indicated by the data, during the time period including the time indicated by the data.

5. The abnormality detection method according to claim 4, wherein
the storage unit stores information indicating a certainty of the monitored subject assuming the predetermined posture in each of the predetermined time periods in each of a presence and an absence of sound equal to or greater than a predetermined sound pressure,
the acquiring includes acquiring data indicating the time and a presence/absence of sound equal to or greater than the predetermined sound pressure, when the monitored subject is detected to have assumed the predetermined posture based on the output value from the sensor, and
the detecting includes referring to the storage unit to detect an abnormality of the monitored subject based on the certainty of the monitored subject assuming the predetermined posture in the presence/absence of the sound indicated by the data, during the time period including the time indicated by the data.

6. The abnormality detection method according to claim 5, wherein
the storage unit stores information indicating a certainty of the monitored subject assuming the predetermined posture in each of the predetermined time periods in each of predetermined movement types,
the acquiring includes acquiring data indicating the time and a movement type when the monitored subject is detected to have assumed the predetermined posture based on the output value from the sensor, and
the detecting includes referring to the storage unit to detect an abnormality of the monitored subject based on the certainty of the monitored subject assuming the predetermined posture by the movement type indicated by the data, during the time period including the time indicated by the data.

7. The abnormality detection method according to claim 6, wherein
the storage unit stores information indicating a certainty of the monitored subject assuming the predetermined posture in each of the predetermined time periods in each of predetermined surrounding environments,
the acquiring includes acquiring data indicating the time and a surrounding environment when the monitored subject is detected to have assumed the predetermined posture based on the output value from the sensor, and
the detecting includes referring to the storage unit to detect an abnormality of the monitored subject based on the certainty of the monitored subject assuming the predetermined posture in the surrounding environment indicated by the data, during the time period including the time indicated by the data.

8. The abnormality detection method according to claim 7, wherein
the surrounding environment is identified by at least any of a temperature, a humidity, an atmospheric pressure, and a wet-bulb globe temperature detected by an output value from the sensor.

9. The abnormality detection method according to claim 8, further comprising:
accumulating, by the computer, data indicative of a posture of the monitored subject, detected by the output value from the sensor and the time when the posture is detected; and
calculating and recording in the storage unit, by the computer, a certainty of the monitored subject assuming the predetermined posture in each of the predetermined time periods, based on the accumulated data.

10. The abnormality detection method according to claim 9, wherein
the data further indicates at least any of a place, a presence/absence of sound equal to or more than the predetermined sound pressure, a movement type, and a surrounding environment when the posture of the monitored subject is detected by the output value from the sensor.

11. The abnormality detection method according to claim 10, wherein
the storage unit stores information indicative of a certainty of the monitored subject assuming the predetermined posture in each of the predetermined time periods in each of predetermined day-of-week classifications, and
the detecting includes referring to the storage unit to detect an abnormality of the monitored subject based on the certainty of the monitored subject assuming the predetermined posture during the time period including the time, in the day-of-week classification including the time.

12. The abnormality detection method according to claim 11, wherein
the storage unit stores information indicative of a certainty of the monitored subject assuming the predetermined posture in each of the predetermined time periods in each of predetermined pulse rate ranges,
the acquiring includes acquiring data indicative of the time and a pulse rate when the monitored subject is detected to assume the predetermined posture based on the output value from the sensor, and
the detecting includes referring to the storage unit to detect an abnormality of the monitored subject based on the certainty of the monitored subject assuming the predetermined posture during the time period including the time, in the pulse rate range including the pulse rate indicated by the data.

13. An abnormality detection program causing a computer to execute a process comprising:
acquiring data indicating a time when a monitored subject is detected to have assumed a predetermined posture, based on an output value from a sensor corresponding to the monitored subject; and
referencing a storage unit storing information identifying a time period when the monitored subject assumes the predetermined posture and detecting an abnormality of the monitored subject when the time indicated by the acquired data is not included in the time period.

14. An information processing apparatus comprising
a control unit configured to:
acquire data indicating a time when a monitored subject is detected to have assumed a predetermined posture, based on an output value from a sensor corresponding to the monitored subject; and
reference a storage unit storing information identifying a time period when the monitored subject assumes the predetermined posture and detect an abnormality of the monitored subject when the time indicated by the acquired data is not included in the time period.
